# EUROPEAN PATENT APPLICATION

(11) **EP 3 889 251 A1**
(43) Date of publication of application: **06.10.2021**
(21) Application number: 20382260.6
(22) Date of filing: 31.03.2020
(51) Int. Cl.: C12N 1/20, C07K 14/30, C07K 16/12, C12N 5/00

(54) **CULTURE MEDIA FOR MYCOPLASMA**

(71) Applicant: Fundació Centre de Regulació Genòmica, 08003 Barcelona (ES); Institució Catalana de Recerca i Estudis Avançats, 08010 Barcelona (ES)
(72) Inventor: BURGOS CASTELLANOS, Raúl, 08110 Montcada i Reixac, Barcelona (ES); LLUCH SENAR, Maria, 08174 Sant Cugat del Vallés, Barcelona (ES); SERRANO PUBUL, Luis, 08950 Esplugues de Llobregat, Barcelona (ES)
(74) Representative: ABG Intellectual Property Law, S.L.

(57) **Abstract**

The invention relates to serum free media for the culture of Mycoplasma as well as to methods for the culture of Mycoplasma using the serum free culture media.

## Description

### FIELD OF THE INVENTION

The invention relates to cell culture medium formulations. Specifically, the invention provides serum-free cell culture medium formulations that facilitate the in vitro cultivation of Mycoplasma cells.

### BACKGROUND OF INVENTION

Mycoplasmas comprise a large group of species capable of colonizing a wide range of organisms, including animals, plants, insects and humans. They are characterized to lack a cell wall, which makes them naturally resistant to antibiotics that target cell wall synthesis, but also more sensitive to membrane-active agents. Mycoplasmas have also reduced genomes with few metabolic capabilities, thus relying on their host for much of their nutrition. For all these reasons, growth of mycoplasma species in axenic conditions has been historically difficult and dependent on animal serum.

Mycoplasmas are generally surface parasites. Some species act as commensal bacteria, living innocuously with their host as part of the natural flora. However, many species like *M. hyopneumoniae* act as pathogens, often causing chronic infections. Indeed, diseases associated to mycoplasmas are an important economic burden, both in human and livestock systems. Vaccination has been proven as an efficient strategy to alleviate the economic impact that some mycoplasma infections cause on milk production, weight gain and animal health. In this regard, some of the most effective vaccines available against mycoplasma infections are live-attenuated or inactivated vaccines, in which growth and production of the bacterial strain is required. The culture medium used in the production of these vaccines usually contains animal components such as BHI (brain heart infusion broth), PPLO (beef heart infusion, pancreatic digest of casein and beef extract broth) and animal serum. The use of animal serum in culture medium entails several disadvantages, including: low batch-to-batch reproducibility, not defined composition, possible interference with downstream processing, and increase considerably the cost of the medium. In addition, both the use of animal serum and raw materials derived from animals have important safety concerns, since these may contain viruses, antibiotics, endotoxins and other bioactive molecules. Therefore, there is a need in the art for the development of a culture medium that: (1) is safe and free of animal components, (2) supports robust growth, and (3) allows serial passaging in mycoplasma vaccine development or other mycoplasma-based therapies.

### SUMMARY OF THE INVENTION

The authors of the invention have developed several culture media that are capable of supporting the growth of Mycoplasma cells and, in particular, of *Mycoplasma hyopneumoniae* cells, to biomass levels similar to those obtained with standard growth medium, said media being characterized in that they are free of animal serum and, in some cases, free of components of animal origin (i.e. bovine seroalbumin, BSA).

In a first aspect, the invention relates to a serum free medium suitable for the culture of *Mycoplasma pneumoniae* that comprises:
(i) a carbon source comprising at least a pyruvate salt,
(ii) an amino acid mixture,
(iii) a vitamin mixture,
(iv) lipids,
(v) inorganic salts,
(vi) a nucleoside mixture comprising thymidine,
(vii) a mixture of enzymatic cofactors,
(viii) a pyruvate salt,
(ix) a proteolytic digest of a microorganism or of a plant or animal tissue and
(x) a vehicle to solubilize and deliver the lipids.

In another aspect, the invention provides a method for the culture of a Mycoplasma strain comprising placing an inoculum of said strain into the media of the invention and maintaining the inoculated media under conditions adequate for the proliferation of the Mycoplasma strain.

In another aspect, the invention provides a method for obtaining a biomass of a Mycoplasma strain which comprises placing an inoculum of said Mycoplasma strain into the media according to the invention and maintaining the inoculated media under conditions adequate for the proliferation of the Mycoplasma strain and the formation of the biomass.

### DESCRIPTION OF THE FIGURES

**Figure 1****.** Composition and biomass yield of different media versions. Table showing the detailed composition of each of the different media versions developed for *M*. *hyopneumoniae* cultivation.
**Figure 2** DNA biomass yield of different media versions after 96h of culture as compare to rich medium.
**Figure 3****.** Scheme showing the workflow during the process of medium optimization.
**Figure 4****.** Impact of several components on DNA biomass yield using a medium free of serum. (A). Fold change in DNA biomass yield after adding new components (BSA: bovine serum albumin replacing cyclodextrin, FA, fatty acids including palmitic and oleic acid, PG: L-α-Phosphatidyl-DL-glycerol, DPPC: 1,2-Dipalmitoyl-sn-glycero-3-phosphocholine) or optimizing original components (SPM: decrease of sphingomyelin from 40 to 10 µg/ml, Vit: removal of vitamin mix including pyridoxamine, nicotinic acid, riboflavin and choline pyridoxamine, Pyr: increase of pyruvate from 2.2g/L to 4g/L).
   (B). Essentiality of several components in medium performance shown as the relative DNA biomass yield compared to the same media formulation after removing the indicated component (CMRL: CMRL-1066 medium CMRL defined medium, TCY: TC Yeastolate, PC: phosphatidylcholine, SPM: sphingomyelin).
**Figure 5****.** Growth curve analysis of *M. hyopneumoniae* non-pathogenic strain J in rich medium (Friss) and serum-free medium versions. Cultures of 30ml in 125ml Erlenmeyer flasks were inoculated with frozen stocks (1:200 dilution) and DNA biomass measured over time during 7 days. Data for each medium represent the mean±SD of 3 biological culture replicates.

### DETAILED DESCRIPTION OF THE INVENTION

The invention relates to a serum-free culture medium for the growing of *Mycoplasma hyopneumoniae.* Said serum-free culture medium comprises at least one carbon source, amino acids, vitamins, amino acids, nucleotides, lipids, inorganic salts enzymatic cofactors, buffering agents, a proteolytic digest of a microorganism, plant or animal tissue and a vehicle to solubilize and deliver the lipids.

Thus in a first aspect, the invention relates to a serum free medium suitable for the culture of *Mycoplasma pneumoniae* that comprises:
(i) a carbon source comprising at least a pyruvate salt,
(ii) an amino acid mixture,
(iii) a vitamin mixture,
(iv) lipids,
(v) inorganic salts,
(vi) a nucleoside mixture comprising thymidine,
(vii) a mixture of enzymatic cofactors,
(viii) a proteolytic digest of a microorganism or of a plant or animal tissue and
(ix) a vehicle to solubilize and deliver the lipids.

As used herein, the term "serum-free medium" refers to medium which is substantially free of serum and which can maintain *in vitro* growth and proliferation of cells for a long period. There is no particular limitation on the serum-free medium of the present invention, which can be any serum-free culture medium suitable for cultivation or preservation of a culture of *Mycoplasma hyopneumoniae.* In some embodiments, the medium according to the present invention can maintain growth of Mycoplasma cells, preferably *M.hyopneumoniae* cells until biomass levels are reached which are at least 10%, at least 20%, at least 30%, at least 40%, at least 50%, at least 60%, at least 70%, at least 80%, at least 90% of the biomass levels that can be reached with rich medium such as, for instance, the Hayflick medium,

The term, "culture", "cell culture" or "cultivation" refers to the maintenance or proliferation of cells in an artificial in vitro environment. The culture media of the present invention can be used for the culture of any Mycoplasma species, especially *Mycoplasma hyopneumoniae.*

The term "Mycoplasma" as used herein, refers to a genus of spherical to filamentous cells with no cell walls. Presently, more than 100 species have been included in the genus Mycoplasma. Mycoplasma are the smallest self-replicating organisms with the smallest genomes, comprising a total of about 500 to 1000 genes. Mycoplasma are nutritionally fastidious. Many require cholesterol, a unique property among prokaryotes.

The term *"Mycoplasma hyopneumoniae"*, as used herein, refers to a species of the Mollicutes which causes the disease the disease porcine enzootic pneumonia. As all Mycoplasma species, also *M. hyopneumoniae* is characterized by the absence of a peptidoglycan cell wall and resulting resistance to many antibacterial agents.

The carbon source in the serum-free culture medium according to the invention comprises pyruvate, preferably in the form of a salt and, more preferably, as a sodium salt.

In one embodiment, the sodium pyruvate is found at a concentration of at least 2.2 g/L, preferably at least 4 g/L. In further embodiments, the sodium pyruvate is found at a concentration of 0,1 g/L to 10 g/L, 0,2 to 8 g/L, 0,4 to 6 g/L.

The carbon source in the serum-free culture medium according to the invention comprises, in addition to a pyruvate salt, a sugar, preferably a monosaccharide such as fructose and glucose, a disaccharide such as lactose and maltose, or a mixture thereof. In an embodiment, the carbon source is selected from glucose, mannose, , acetate, glucuronate, pyruvate, acetate, ascorbate and lactate. In another embodiment, the carbon source is mannose or glucose.

A carbon source suitable for the serum-free culture medium according to the invention preferably is or comprises glucose. The carbon source in the serum-free culture medium according to the invention preferably is present between 0.01 and 30 % (w/w), more preferably between 0.02 and 2% (w/w).

In some embodiments, the carbon source is provided in combination with a second polyol compound, such as glycerol. When present, glucose and glycerol preferably are present between 0.2 and 25% (w/w), more preferably between 0.5 and 2% (w/w), preferably at 0,75% (w/w) for glucose; and between 0.01 and 0.5% (w/w), more preferably between 0.002 and 0.5% (w/w), more preferably between 0.005 and 0.05% (w/w), preferably at 0.025% (w/w) for glycerol.

In a preferred embodiment, the glycerol concentration is of at least 0.01% (w/w), preferably of at least 0.025% (w/w) and more preferably of at least 0,05% (w/w).

In a particular embodiment the source of carbon is D-glucose in a concentration of between 100 to 5000 mg/L. In another embodiment, the concentration of D-glucose is of about 500 mg/L. In another embodiment the concentration of D-glucose is of about 2000 mg/L.

The chemically defined medium comprises one or more of the amino acids. The term "one or more amino acids" refers to native amino acids or their derivatives (e.g., amino acid analogs), as well as their D- and L-forms.

In a preferred embodiment, the serum-free culture medium according to the invention comprises at least the amino acids Glycine, hydroxy L-proline, L-Alanine, L-Arginine hydrochloride, L-Aspartic acid, L-Cysteine, L-Cystine, L-Glutamic Acid, L-Glutamine, L-Histidine hydrochloride-H2O, L-Isoleucine, L-Leucine, L-Lysine hydrochloride, L-Methionine, L-Phenylalanine, L-Proline, L-Serine, L-Threonine, L-Tryptophan, L-Tyrosine, L-Valine.

In a preferred embodiment, the serum-free culture medium of the invention comprises the amino acids Glycine, Hydroxy L-proline, L-Alanine, L-Arginine hydrochloride, L-Cysteine, L-Cystine, L-Glutamine, L-Histidine hydrochloride-H₂O, L-Isoleucine, L-Leucine, L-Lysine hydrochloride, L-Methionine, L-Phenylalanine, L-Proline, L-Serine, L-Threonine, L-Tryptophan, L-Tyrosine, L-Valine but lacks L-Aspartic acid, L-Glutamic Acid and L-asparagine.

In a preferred embodiment, the serum-free culture medium of the invention comprises the amino acids Glycine, Hydroxy L-proline, L-Alanine, L-Arginine hydrochloride, L-Cysteine, L-Cystine, L-Glutamine Acid, L-Histidine hydrochloride-H₂O, L-Isoleucine, L-Leucine, L-Lysine hydrochloride, L-Methionine, L-Phenylalanine, L-Proline, L-Serine, L-Threonine, L-Tryptophan, L-Tyrosine, L-Valine but lacks L-Aspartic acid.

When present in the serum-free culture medium, the amino acids are preferably contained in the concentration ranges defined below.

Glycine in a range of 0.1 mg/L to 1000 mg/L, more preferably in a range between 1 mg/L to 500mg/L, preferably between 10 to 200 mg/L. In a particular embodiment the concentration of Glycine is of about 25 mg/L. In another embodiment, the concentration of glycine if of about 50 mg/L. In another embodiment, the concentration of glycine is of about 100 mg/L.

Hydroxy-L-proline in a range between 0.01 mg/L to 200 mg/L, more preferably between 0.1 mg/L to 100 mg/L. in a particular embodiment, the concentration of Hydroxy-L-proline is of about 1 mg/L. In another embodiment, the concentration of Hydroxy-L-proline is of about 10 mg/L. In another embodiment, the concentration of Hydroxy-L-proline is of about 20 mg/L.

L-alanine in a range of 0.1 mg/L to 1000 mg/L. more preferably between 1 mg/L to 100 mg/L. In a particular embodiment, the concentration of L-alanine is of about 12 mg/L. In another embodiment, the concentration of L-alanine is of about 25 mg/L. In another embodiment the concentration of L-alanine is of about 50 mg/L.

L-arginine hydrochloride in a range of 0.1 mg/L to 1000 mg/L, more preferably between 1 mg/L to 500 mg/L. In a particular embodiment, the concentration of L-arginine hydrochloride is of about 35 mg/L. In another embodiment, the concentration of L-arginine hydrochloride is of about 70 mg/L. In another embodiment, the concentration of L-arginine hydrochloride is of about 140 mg/L.

L-aspartic acid in a range of 0.1 mg/L to 1000 mg/L, more preferably between 1 mg/L to 100 mg/L. In a particular embodiment, the concentration of L-aspartic acid is of about 15 mg/L. In another embodiment, the concentration of L-aspartic acid is of about 30 mg/L. In another embodiment, the concentration of L-aspartic acid is of about 60 mg/L.

L-cysteine in a range of 1 mg/L to 1000 mg/L, more preferably between 50 mg/L to 500. In a particular embodiment, the concentration of L-cysteine is about 100 mg/L. In another embodiment, the concentration of L-cysteine is of about 200 mg/L. In another embodiment, the concentration of L-cysteine is of about 400 mg/L.

L-cystine in a range of 0.1 mg/L to 1000 mg/L, more preferably between 1 mg/L to 100 mg/L. In a particular embodiment, the concentration of L-cystine is of about 10 mg/L. In another embodiment, the concentration of L-cystine is of about 20 mg/L. In another embodiment, the concentration of L-cystine is of about 40 mg/L.

L-glutamic acid in a range of 0.1 mg/L to 1000 mg/L, more preferably between 1 mg/L to 500 mg/L. In a particular embodiment, the concentration of L-glutamic acid is of about 35 mg/L. In another embodiment, the concentration of L-glutamic acid is of about 15 mg/L. In another embodiment, the concentration of L-glutamic acid is of about 15 mg/L , preferably at 150 mg/L.

L-histidine hydrochloride monohydrate in a range of 0.1 mg/L to 500 mg/L, more preferably between 1 mg/L to 100 mg/L. In a particular embodiment, the concentration of L-histidine hydrochloride monohydrate is of about 10 mg/L. In another embodiment, the concentration of L-histidine hydrochloride monohydrate is of about 20 mg/L. In another embodiment, the concentration of L-histidine hydrochloride monohydrate is of about 40 mg/L.

L-isoleucine in a range of 0.1 mg/L to 1000 mg/L, more preferably between 1 mg/L to 100 mg/L. In a particular embodiment, the concentration of L-isoleucine is of about 10 mg/L. In another embodiment, the concentration of L-isoleucine is of about 20 mg/L. In another embodiment, the concentration of L-isoleucine is of about 40 mg/L.

L-leucine in a range of 0.1 mg/L to 1000 mg/L, more preferably between 1 mg/L to 500 mg/L. In a particular embodiment, the concentration of L-leucine of about 30 mg/L. In another embodiment, the concentration of L-leucine is of about 60 mg/L. In another embodiment, the concentration of L-leucine is of about 120 mg/L.

L-lysine hydrochloride in a range of 0.1 mg/L to 1000 mg/L, more preferably between 1 mg/L to 500 mg/L. In a particular embodiment, the concentration of L-lysine hydrochloride is of about 35 mg/L. In another embodiment, the concentration of L-lysine hydrochloride is of about 70 mg/L. In another embodiment, the concentration of L-lysine hydrochloride is of about 140 mg/L.

L-methionine in a range of 0.1 mg/L to 1000 mg/L, more preferably between 1 mg/L to 100 mg/L. In a particular embodiment, the concentration of L-methionine is of about 7.5 mg/L. In another embodiment, the concentration of L-methionine is of about 15 mg/L. In another embodiment, the concentration of L-methionine is of about 30 mg/L.

L-phenylalanine in a range of 0.1 mg/L to 1000 mg/L, more preferably between 1 mg/L to 100 mg/L. In a particular embodiment, the concentration of L-phenylalanine is of about 12.5 mg/L. In another embodiment, the concentration of L-phenylalanine is of about 25 mg/L. In another embodiment, the concentration of L-phenylalanine is of about 50 mg/L.

L-proline in a range of 0.1 mg/L to 1000 mg/L, more preferably between 1 mg/L to 100 mg/L. In a particular embodiment, the concentration of L-proline is of about 20 mg/L. In another embodiment, the concentration of L-proline is of about 40 mg/L. In another embodiment, the concentration of L-proline is of about 80 mg/L.

L-serine in a range of 0.1 mg/L to 1000 mg/L, more preferably between 1 mg/L to 100 mg/L. In a particular embodiment, the concentration of L-serine is of about 12.5 mg/L. In another embodiment, the concentration of L-serine is of about 25 mg/L. In another embodiment, the concentration of L-serine is of about 50 mg/L.

L-threonine in a range of 0.1 mg/L to 1000 mg/L, more preferably between 1 mg/L to 100 mg/L. In a particular embodiment, the concentration of L-threonine is of about 15 mg/L. In another embodiment, the concentration of L-threonine is of about 30 mg/L. In another embodiment, the concentration of L-threonine is of about 60 mg/L.

L-tryptophan in a range of 0.1 mg/L to 1000 mg/L, more preferably between 1 mg/L to 1000 mg/L. In a particular embodiment, the concentration of L-tryptophan is of about 5 mg/L. In another embodiment, the concentration of L-tryptophan is of about 10 mg/L. In another embodiment, the concentration of L-tryptophan is of about 20 mg/L.

L-tyrosine in a range of 0.1 mg/L to 1000 mg/L, more preferably between 1 mg/L to 100mg/L. In a particular embodiment, the concentration of L-tyrosine is of about 20 mg/L. In another embodiment, the concentration of L-tyrosine is of about 40 mg/L. In another embodiment, the concentration of L-tyrosine is of about 80 mg/L.

L-valine in a range of 0.1 mg/L to 1000 mg/L, more preferably between 1 mg/L to 100 mg/L. In a particular embodiment, the concentration of L-valine is of about 12.5 mg/L. In another embodiment, the concentration of L-valine is of about 25 mg/L. In another embodiment, the concentration of L-valine is of about 50 mg/L.

The serum-free culture medium additionally comprises L-Glutamine. In a preferred embodiment, the content of the serum-free medium in L-Glutamine is of at least 1 nM, more preferably of at least 2 mM.

Glutamine is known to be quite unstable in an aqueous solution. As been reported, glutamine may be stabilized with one or more divalent cations selected from the group consisting of calcium and magnesium, whereby the ration of glutamine to divalent cations is in the range of from 2:1 to 2.9:1 (US patent 5,474,931). In addition, the presence of pantothenate and sodium chloride may help stabilize glutamine and prevent its degradation into pyrrolidone carboxylic acid and ammonia. As an alternative, or in addition, unstable glutamine may be replaced with the dipeptide I-alanyl-I-glutamine or glycyl-I-glutamine. Thus in an embodiment, the content of the I-alanyl-I-glutamine or glycyl-I-glutamine in the free-serum culture medium is of at least 1 mM, more preferably, of at least 2 mM.

Similarly, redox active cysteine may be replaced with N-acetyl-I-cysteine of S-sulfo-I-cysteine (Hecklau et al., 2016, J. Biotech 218: 53-63), and the amino acid derivative phosphor-I-tyrosine may replace tyrosine (Zimmer et al., 2014, J. Biotechnol 186: 110-8).

In another embodiment, the essential amino acids or the serum-free culture medium are preferentially administered in the form of peptides. Suitable peptides for the serum-free medium or the invention include without limitation, dipeptides selected from the group consisting of L-alanyl-L-glutamine, glycyl-L-glutamine and N-acetyl-L-glutamine. If the amino acids are provided in the form of peptides, then the culture medium may further comprise a reducing or antioxidant agent so as to avoid the formation of disulphide bridges between the Cys residues. Suitable antioxidant agents and concentrations thereof are defined below in the context of the antioxidant components. In a preferred embodiment, the antioxidant is reduced glutathione.

In a particular embodiment, the peptides are derived from the proteolytic digest of a microorganism or of a plant or animal tissue, in which case the peptide may be selected from peptone, PPLO and yeastolate, as will be specified below.

The serum-free culture medium of the invention comprises a vitamin mixture, said vitamin mixture comprising thioctic acid. In addition to the thioic acid, the vitamin mixture preferably includes ascorbic acid, biotin, Choline chloride, D-Calcium pantothenate, Folic Acid, Niacinamide, Nicotinic acid (Niacin), Para-Aminobenzoic Acid, vitamin B6, Pyridoxal hydrochloride, Pyridoxine hydrochloride, Riboflavin and Thiamine hydrochloride. These vitamins and/or essential nutrients can be obtained commercially, for example from Sigma (Sant Louis, Missouri). Said vitamins optionally may be provided by addition of a yeast extract, as it is known by a person skilled in the art.

The term "vitamin" or "essential nutrient" as used herein refers to an organic molecule (or related set of molecules) that is an essential micronutrient that an organism needs in small quantities for the proper functioning of its metabolism. Essential nutrients cannot be synthesized in the organism, either at all or not in sufficient quantities, and therefore must be obtained through the diet. The term vitamin does not include the three other groups of essential nutrients: minerals, essential fatty acids, and essential amino acids.

The term "Ascorbic acid" or "vitamin C", as used herein refers to a natural water soluble vitamin of formula C₆H₈O₆ or HC₆H₇O₆. Ascorbic acid is a potent reducing and antioxidant agent that functions in fighting bacterial infections, in detoxifying reactions, and in the formation of collagen in fibrous tissue, teeth, bones, connective tissue, skin, and capillaries.

As used herein, the term "biotin", also known as vitamin H, vitamin B7 or coenzyme R, is an enzyme cofactor of formula C₁₀H₁₆N₂O₃S. It occurs mainly bound to proteins or polypeptides and is abundant in liver, kidney, pancreas, yeast, and milk. Biotin has been recognized as an essential nutrient.

The term "Choline chloride", also known as hepacholine, biocolina, 2-Hydroxy-N,N,N-trimethylethanaminium, refers to a quaternary ammonium salt with choline cation and chloride anion, of formula C₅H₁₄NO.Cl or C₅H1₄ClNO. It has a role as an animal growth promotant. It is a chloride salt and a quaternary ammonium salt. It contains a choline.

As used herein, the term "D-Calcium pantothenate", also known as calpanate refers to the calcium salt of the water soluble vitamin B5, ubiquitously found in plants and animal tissues with antioxidant property. Pentothenate is a component of coenzyme A (CoA) and a part of the vitamin B2 complex. Vitamin B5 is a growth factor and is essential for various metabolic functions, including the metabolism of carbohydrates, proteins, and fatty acids. This vitamin is also involved in the synthesis of cholesterol, lipids, neurotransmitters, steroid hormones, and hemoglobin.

The term "Folic Acid", as used herein, also known as folate or vitamin M, of formula C₁₉H₁₉N₇O₆, is a collective term for pteroylglutamic acids and their oligoglutamic acid conjugates. As a natural water-soluble substance, folic acid is involved in carbon transfer reactions of amino acid metabolism, in addition to purine and pyrimidine synthesis, and is essential for hematopoiesis and red blood cell production.

The term "Nicotinamide", also known as niacinamide or 3-Pyridinecarboxamide is the active form of vitamin B3 and a component of the coenzyme nicotinamide adenine dinucleotide (NAD). It has the molecular formula C₆H₆N₂O.

The term "Nicotinic acid", also known as niacin, or vitamin B3, with molecular formula C₆H₅NO₂ or HOOC₅H₄N or C₅H₄NCOOH, is a water-soluble vitamin whose derivatives such as NADH, NAD, NAD+, and NADP play essential roles in energy metabolism in the living cell and DNA repair. The designation vitamin B3 also includes the amide form, nicotinamide or niacinamide.

In some embodiments, the medium contains nicotinic acid at a concentration of at least 0,2 µg/ml, preferably 0,5 µg/ml.

The term "Para-Aminobenzoic Acid", also known as 4-aminobenzoic acid, or simply PABA, is an organic compound with molecular formula C₇H₇NO₂. PABA is a white crystalline substance that is only slightly soluble in water. It consists of a benzene ring substituted with an amino group and a carboxylic acid. PABA is an essential nutrient for some bacteria and is sometimes called Vitamin Bx.

As used herein, the term "vitamin B6" refers to a refers to a group of chemically similar compounds which can be interconverted in biological systems to yield the active form which is, pyridoxal 5'-phosphat. The term includes pyridoxal hydrochloride, pyridoxine hydrochloride and pyridoxamine.

As used herein, the term "Pyridoxal hydrochloride", also known as Pyridoxal HCI, or 3-Hydroxy-5-(hydroxymethyl)-2-methylisonicotinaldehyde hydrochloride, with molecular formula C₈H₁₀ClNO₃, refers to a hydrochloride obtained by combining pyridoxal with one molar equivalent of hydrochloric acid. It has a role as an Escherichia coli metabolite, a Saccharomyces cerevisiae metabolite, a cofactor, a human metabolite and a mouse metabolite. It is a hydrochloride and a pyridinium salt. It contains a pyridoxal(1+).

The term "Pyridoxine hydrochloride", as used herein, refers to the compound of formula C₈H₁₂ClNO₃, which is the hydrochloride salt form of pyridoxine, a water-soluble vitamin B.

The term pyridoxamine, as used herein, refers to one form of vitamin B6 which consists of a pyridine ring structure, with hydroxyl, methyl, aminomethyl, and hydroxymethyl substituents. It differs from pyridoxine by the substituent at the 4-position, which is an aminomethyl group in the pyridoxamine and a hydroxymethyl group in pyridoxine

The term "Riboflavin", as used herein, also known as lactoflavin or vitamin B2, refers to is an essential human nutrient that is a heat-stable and water-soluble flavin belonging to the vitamin B family. Riboflavin is a precursor of the coenzymes flavin mononucleotide (FMN) and flavin adenine dinucleotide (FAD). It has the molecular formula C₁₇H₂₀N₄O₆.

The term "Thiamine hydrochloride", as used herein, also known as Thiamine HCl, Aneurine hydrochloride or Vitamin B1 hydrochloride, refers to the hydrochloride salt form of thiamine, a vitamin essential for aerobic metabolism, cell growth, transmission of nerve impulses and acetylcholine synthesis. Upon hydrolysis, thiamine hydrochloride is phosphorylated by thiamine diphosphokinase to form active thiamine pyrophosphate (TPP), also known as cocarboxylase. TPP is a coenzyme for many enzymatic activities involving fatty acid, amino acid and carbohydrate metabolism. It has the molecular formula HC₁₂H₁₇ON₄SCl₂ or C₁₂H₁₈Cl₂N₄OS.

As used herein the term "thioctic acid" also known as "Lipoic acid (LA)" or "alpha lipoic acid (ALA)" refers to the compound of formula (R)-5-(1,2-Dithiolan-3-yl)pentanoic acid and is an organosulfur compound derived from octanoic acid. Thioctic acid contains two sulfur atoms (at C6 and C8) connected by a disulfide bond and is thus considered to be oxidized although either sulfur atom can exist in higher oxidation states.

In a preferred embodiment the serum-free culture medium of the invention comprises thioctic acid at a concentration in a range between 0.001 mg/L to 10 mg/L, more preferably between 0.01 to 1 mg/L, preferably at 0.1 mg/L. In another embodiment, the serum-free culture medium of the invention comprises thioctic acid at a concentration of at least 0,1 µg/mL, preferably 0,2 µg/mL.

The vitamin mixture preferably contains the following vitamins in the concentration ranges defined below:
- Thioctic acid, in a range between 0.001 mg/L to 10 mg/L, more preferably between 0.01 to 1 mg/L, preferably at 0.1 mg/L. In one embodiment, the thioctic acid is present at a concentration of at least 0,1 µg/ml, preferably of at least 0,2 µg/ml,
- Ascorbic acid in a range between 0.1 to 1000 mg/L, more preferably between 1 to 100 mg/L. In a particular embodiment, the concentration of ascorbic acid is of about 25 mg/L. In another embodiment, the concentration of ascorbic acid is of about 50 mg/L. In another embodiment, the concentration of ascorbic acid is of about 100 mg/L.
- Biotin in a range between 0.0001 to 1 mg/ml, preferably between 0.001 to 0.1 mg/L. In a particular embodiment, the concentration of biotin is of about 0.005 mg/L. In another embodiment, the concentration of biotin is of about 0.01 mg/L. In another embodiment, the concentration of biotin is of about 0.02 mg/L.
- Choline chloride in a range between 0.001 to 10 mg/L, more preferably between 0.01 to 1 mg/L. In a particular embodiment, the concentration of Choline chloride is of about 0.25 mg/L. In another embodiment, the concentration of Choline chloride is of about 0.5 mg/L. In another embodiment, the concentration of Choline chloride is of about 1 mg/L. In another embodiment, choline is present at a concentration of at least 0,2 µg/ml, preferably of at least 0,5 µg/ml.
- D-Calcium pantothenate in a range between 0.0001 to 1 mg/L, preferably between 0.001 to 0.1 mg/L. In a particular embodiment, the concentration of D-Calcium pantothenate is of about 0.005 mg/L. In another embodiment, the concentration of D-Calcium pantothenate is of about 0.01 mg/L. In another embodiment, the concentration of D-Calcium pantothenate is of about 0.02 mg/L.
- Folic acid in a range between 0.0001 to 1 mg/L, preferably between 0.001 to 0.1 mg/L, preferably at 0.01 mg/L. In a particular embodiment, the concentration of Folic acid is of about 0.005 mg/L. In another embodiment, the concentration of Folic acid is of about 0.01 mg/L. In another embodiment, the concentration of Folic acid is of about 0.02 mg/L.
- Niacinamide in a range between 0.0001 to 1 mg/L, preferably between 0.001 to 0.1 mg/L. In a particular embodiment, the concentration of Niacinamide is of about 0.0125 mg/L. In another embodiment, the concentration of Niacinamide is of about 0.025 mg/L. In another embodiment, the concentration of Niacinamide is of about 0.05 mg/L.
- Nicotinic acid (Niacin) in a range between 0.0001 to 1 mg/L, preferably between 0.001 to 0.1 mg/L. In a particular embodiment, the concentration of Nicotinic acid is of about 0.0125 mg/L. In another embodiment, the concentration of Nicotinic acid is of about 0.025 mg/L. In another embodiment, the concentration of Nicotinic acid is of about 0.05 mg/L. In one embodiment, the nicotinic acid is present at a concentration of at least 0,2 µg/ml, preferably of about 0,5 µg/ml or of at least 0,5 µg/ml.
- Para-Aminobenzoic acid in a range between 0.0001 to 1 mg/L, preferably between 0.001 to 0.1 mg/L. In a particular embodiment, the concentration of Para-Aminobenzoic acid is of about 0.025 mg/L. In another embodiment, the concentration of Para-Aminobenzoic acid is of about 0.05 mg/L. In another embodiment, the concentration of Para-Aminobenzoic acid is of about 0.1 mg/L.
- Pyridoxal-hydrochloride in a range between 0.0001 to 1 mg/L, preferably between 0.001 to 0.1 mg/L. In a particular embodiment, the concentration of Pyridoxal-hydrochloride is of about 0.0125 mg/L. In another embodiment, the concentration of Pyridoxal-hydrochloride is of about 0.025 mg/L. In another embodiment, the concentration of P Pyridoxal-hydrochloride is of about 0.05 mg/L.
- Pyridoxine hydrochloride in a range between 0.0001 to 1 mg/L, preferably between 0.001 to 0.1 mg/L. In a particular embodiment, the concentration of Pyridoxine hydrochloride is of about 0.0125 mg/L. In another embodiment, the concentration of Pyridoxine hydrochloride is of about 0.025 mg/L. In another embodiment, the concentration of Pyridoxine hydrochloride is of about 0.05 mg/L.
- Riboflavin in a range between 0.0001 to 1 mg/L, preferably between 0.001 to 0.1 mg/L. In a particular embodiment, the concentration of Riboflavin hydrochloride is of about 0.005 mg/L. In another embodiment, the concentration of Riboflavin is of about 0.01 mg/L. In another embodiment, the concentration of Riboflavin is of about 0.02 mg/L. In further embodiments, riboflavin is present at a concentration of at least 0,2 µg/ml, preferably 0,5 µg/ml.
- Thiamine hydrochloride in a range between 0.0001 to 1 mg/L, preferably between 0.001 to 0.1 mg/L. In a particular embodiment, the concentration of Thiamine hydrochloride is of about 0.005 mg/L. In another embodiment, the concentration of Thiamine hydrochloride is of about 0.01 mg/L. In another embodiment, the concentration of Thiamine hydrochloride is of about 0.02 mg/L.

The serum-free culture medium of the present invention may additionally comprise i-inositol in a range between 0.0001 to 1 mg/L, preferably between 0.001 to 0.1 mg/L, preferably at 0.05 mg/L. In a particular embodiment, the concentration of i-inositol is of about 0.025 mg/L. In another embodiment, the concentration of i-inositol is of about 0.05 mg/L. In another embodiment, the concentration of i-inositol is of about 0.1 mg/L.

The serum-free culture medium of the invention comprises lipids, said lipids being a mixture comprising cholesterol, sphingomyelin or ceramide, phosphatidylcholine, L-α-Phosphatidyl-DL-glycerol, 1,2-Dipalmitoyl-sn-glycero-3-phosphocholine.

The term cholesterol as used herein refers to the chemical compound with the molecular formula C₂₇H₄₆O. Cholesterol is an animal sterol found in the body tissues (and blood plasma) of vertebrates. It is a cholestanoid consisting of cholestane having a double bond at the 5,6-position as well as a 3beta-hydroxy group. It is a 3beta-sterol, a cholestanoid, a C27-steroid and a 3beta-hydroxy-Delta(5)-steroid

In one embodiment, cholesterol is present at a concentration of about 30 µg/mL In further embodiments, cholesterol is present at a concentration of between 1 and 100 µg/mL, 2 and 90 µg/mL, 5 and 80 µg/mL, 10 and 70 µg/mL, 15 and 60 µg/mL, 20 and 50 µg/mL, 25 and 35 µg/mL.

The term "sphingomyelin" (SPM), or formula (E,2S,3R)-3-hydroxy-2-[[(Z)-tetracos-15-enoyl]amino]octadec-4-enyl] 2-(trimethylazaniumyl)ethyl phosphate), is a type of sphingolipid found in animal cell membranes, especially in the membranous myelin sheath that surrounds some nerve cell axons. It usually consists of phosphocholine and ceramide, or a phosphoethanolamine head group; therefore, sphingomyelins can also be classified as sphingophospholipids.

The term "ceramide" as used herein, refers to a family of compounds which result from esterification of sphingosine by a fatty acid. Depending on the type of fatty acid forming part of the ceramide, this can be classified as a Ceramide I, ceramide Ila, ceramide IIb, ceramide III, ceramide IV, ceramide V, ceramide V or ceramide VI).

In one embodiment, sphingomyelin is present at a concentration of about 10 µg/mL. In further embodiments, sphingomyelin is present at a concentration of between 0,1 and 100 µg/mL, 0,5 and 90 µg/mL, 1 and 80 µg/mL, 1,5 and 70 µg/mL, 2 and 60 µg/mL, 3 and 50 µg/mL, 3,5 and 35 µg/mL, 4 and 30 µg/mL, 5 and 20 µg/mL or 5 and 15 µg/mL,

The term "phosphatidylcholine", as used herein refers to a class of phospholipids that have choline as a head group. This phospholipid is composed of a choline head group and glycerophosphoric acid, with a variety of fatty acids. Usually, one is a saturated fatty acid (in the given figure, this can be palmitic or hexadecanoic acid, H₃C-(CH₂)₁₄-COOH; margaric acid identified by Gobley in egg yolk, or heptadecanoic acid H₃C-(CH₂)₁₅-COOH, also belong to that class); and the other is an unsaturated fatty acid (here oleic acid, or 9Z-octadecenoic acid, as in Gobley's original egg yolk lecithin). However, there are also examples of disaturated species.

In one embodiment, phosphatidylcholine is present at a concentration of about 60 µg/mL. In further embodiments, phosphatidylcholine is present at a concentration of between 0,1 and 500 µg/mL, 0,5 and 400 µg/mL, 1 and 300 µg/mL, 5 and 200 µg/mL, 10 and 100 µg/mL, 40 and 70 µg/mL

As used herein, L-α-Phosphatidyl-DL-glycerol refers to a family of compounds in which the DL-glycerol 3-phosphate backbone ester-is bonded to either saturated or unsaturated fatty acids on carbons 1 and 2.

In further embodiments, L-α-Phosphatidyl-DL-glycerol is present at concentrations of between 0,1 and 200 µg/mL, between 0,5 and 150 µg/mL, between 1 and 100 µg/mL, between 0,5 and 50 µg/mL or between 10 and 20 µg/mL.

The term 1,2-Dipalmitoyl-sn-glycero-3-phosphocholine is used to refer to a compound which results from the attachment of two palmitic acids to a phosphatidylcholine head-group.

In further embodiments, the 1,2-Dipalmitoyl-sn-glycero-3-phosphocholine is present at concentrations of between 0,1 and 200 µg/mL, between 0,5 and 150 µg/mL, between 1 and 100 µg/mL, between 0,5 and 50 µg/mL or between 10 and 20 µg/mL

One or more of the lipids mentioned above may be provided as a complex with a delivery vehicle. In one embodiment, cholesterol is provided as a complex with a delivery vehicle. In another embodiment, sphingomyelin is provided as a complex with a delivery vehicle. In another embodiment, phosphatidylcholine is provided as a complex with a delivery vehicle. In another embodiment, L-α-Phosphatidyl-DL-glycerol is provided as a complex with a delivery vehicle. In another embodiment, 1,2-Dipalmitoyl-sn-glycero-3-phosphocholine is provided as a complex with a delivery vehicle. In some embodiments, at least two, at least three, at least four or all lipids defined above are provided as a complex with a delivery vehicle.

It will be understood that the concentrations of these components, while being present at concentration ratios as defined above, should also be present at concentrations that do not result in a substantially toxicity to the cells.

The term "concentration that does not result in a substantial toxicity to the cells" as used herein, refers to the concentration of a component in a culture medium that does not substantially delay the growth of the cells in comparison with the growth in the same medium and under the same condition in the absence of the component. The concentration of a given component that does not result in a substantial toxicity to the cells prevent the cells to grow when present in a culture medium can determined by calculating the IC50 for said component, said IC50 being the concentration that causes a 50% growth inhibition. In some embodiments, the concentration that does not result in a substantial toxicity to the cells for sphingomyelin and for phosphatidylcholine corresponds to the IC50, to 90% of the IC50, to 80% of the IC50, to 70% of the IC50, to 60% of the IC50 or less.

In another embodiment the lipid mixture additionally comprises a fatty acid.

The term "fatty acid" as used herein, refers to an aliphatic carboxylic acid having the formula RCOOH wherein R is an aliphatic group having at least 4 carbons, typically between about 4 and about 28 carbon atoms. The aliphatic R group can be saturated or unsaturated, branched or unbranched. Unsaturated fatty acids" may be monounsaturated or polyunsaturated.

In one embodiment, the fatty acid is palmitic acid, oleic acid or a mixture thereof.

In particular the concentration of palmitic acid in the serum-free culture medium is in the range between 0.01 and 100 mg/L, more preferably between 0.1 and 100 mg/L, preferably between 10 and 40 mg/L, preferably at about 20 mg/L, and more particularly at about 16.6 mg/L.

In another embodiment, the concentration of oleic acid in the serum-free culture medium is in the range between 0.01 and 100 mg/L, more preferably between 0.1 and 100 mg/L (w/w), preferably between 10 and 40 mg/L preferably at about 20 mg/L (w/w), and more particularly at 20 mg/L..

The fatty acids are preferably provided in free form, i.e. not forming part of a complex to a vehicle or a carrier, including bovine serum albumin (BSA), preferably delipidated BSA, polyvinyl alcohol (PVA) or lipid vesicles. In particular embodiment, the carrier to deliver cholesterol is BSA, at a concentration range between 0.1 and 10%, more preferably between 1 and 5%, preferably at about 3%.

The serum-free culture medium also comprises inorganic salts. In an embodiment, the inorganic salt is selected from the group comprising Calcium Chloride (CaCl₂-2H₂O), Magnesium Sulfate (MgSO₄-7H₂O), Potassium Chloride (KCl), Sodium Bicarbonate (NaHCO₃), Sodium Chloride (NaCl), Sodium Phosphate monobasic (NaH₂PO₄-2H₂O).

The inorganic salts are preferably contained in the medium in the concentration ranges defined below.
- Calcium Chloride (CaCl₂-2H₂O), in a range between 1 to 1000 mg/L, more particularly between 10 to 600 mg/L. In an embodiment, the concentration of Calcium Chloride is of about 130 mg/L. In another embodiment, the concentration of Calcium Chloride is about 165 mg/L. In another embodiment, the concentration of Calcium Chloride is about 530 mg/L.
- Magnesium Sulfate (MgSO₄-7H₂O), in a range between 1 to 1000 mg/L, more particularly between 10 to 600 mg/L. In a particular embodiment, the concentration of Magnesium Sulfate is of about 100 mg/L. In another embodiment, the concentration of Magnesium Sulfate is of about 400 mg/L. In another embodiment, the concentration of Magnesium Sulfate is of about 200 mg/L.
- Potassium Chloride (KCl), in a range between 1 and 15000 mg/L, more particularly between 100 to 1000 mg/L. In an embodiment the concentration of Potassium Chloride is of about 200 mg/L. In another embodiment the concentration of Potassium Chloride is of about 400 mg/L. In another embodiment, the concentration of Potassium Chloride is of about 800 mg/L.
- Sodium Bicarbonate (NaHCO₃), in a range between 100 and 10000 mg/L, preferably between 500 and 5000 mg/L. In a particular embodiment, the concentration of Sodium Bicarbonate is of about 1100 mg/L. In another embodiment, the concentration of Sodium Bicarbonate is of about 2200 mg/L, In another embodiment, the concentration of Sodium Bicarbonate is of about 4400 mg/L.
- Sodium Chloride (NaCl), in a range between 100 and 20000 mg/L, preferably between 1000 and 15000 mg/L. In a particular embodiment, the concentration of Sodium Chloride is of about 3400 mg/L. In another embodiment, the concentration of Sodium Chloride is of about 6800 mg/L. In another embodiment, the concentration of Sodium Chloride is of about 13600 mg/L.

In a particular embodiment, the inorganic salts present in the serum-free culture medium of the invention include at least one salt wherein the cation is calcium, magnesium, potassium or sodium and wherein the anion is chloride, sulfate, bicarbonate or phosphate monobasic.

The serum-free culture medium of the invention comprises nucleosides or nucleotides monophosphate. Nucleosides are molecules consisting of a nitrogenous base and either ribose or deoxyribose. Nucleotides monophosphate are molecule consisting of a nucleoside and one phosphate group.

Nucleosides include cytidine, deoxycytidine, uridine, adenosine, deoxyadenosine, guanosine, deoxyguanosine and thymidine.

Nucleotides monophosphate include adenosine monophosphate, guanosine monophosphate, cytidine monophosphate, thymidine monophosphate and uridine monophosphate. Said nucleotides preferably include adenosine ribonucleotide monophosphate, and adenosine deoxyribonucleotide monophosphate, collectively termed herein adenine; guanosine ribonucleotide monophosphate and guanosine deoxyribonucleotide monophosphate, collectively termed herein guanine; cytidine ribonucleotide monophosphate, termed cytidine; cytidine deoxyribonucleotide monophosphate, termed deoxycytidine; thymidine deoxyribonucleotide monophosphate, termed thymidine; uridine ribonucleotide monophosphate, termed uracil; adenosine ribonucleotide 3'5'-biphosphate, or a combination thereof. These nucleosides and nucleotides monophosphate may be obtained commercially, for example from Sigma (Saint Louis, Missouri).

Said nucleotides monophosphate or nucleosides as defined above are preferably present in the serum-free culture medium of the invention at a concentration between 1 µM and 1mM, more preferably at a concentration between 50 µM and 200 µM.

In an embodiment, the serum-free culture medium of the invention comprises nucleosides selected from the group comprising 2'-deoxyadenosine, 2'-deoxycytidine and 2'-deoxyguanosine but lacks thymidine and uridine.

In an embodiment, the serum-free culture medium of the invention comprises nucleosides selected from the group comprising adenosine, cytidine and guanosine but lacks thymidine and uridine.

In an embodiment, the serum-free culture medium of the invention comprises nucleotides monophosphate selected from the group comprising 2'-deoxyadenosine monophosphate, 2'-deoxycytidine monophosphate, 2'-deoxyguanosine monophosphate but lacks thymidine monophosphate and uridine-5'-monophosphate.

In an embodiment, the serum-free culture medium of the invention comprises nucleotides monophosphate selected from the group comprising adenosine monophosphate, cytidine monophosphate, guanosine monophosphate, but lacks thymidine monophosphate and uridine-5'-monophosphate.

These nucleosides and nucleotides monophosphate are preferably contained in the medium in the concentration ranges defined below.

2'-deoxyadenosine or 2'-deoxyadenosine monophosphate can be found in a range between 0.1 and 100 mg/L, preferably between 1 and 100 mg/L. In a particular embodiment, the concentration of 2'Deoxyadenosine or 2'-deoxyadenosine monophosphate is of about 5 mg/L. In another embodiment, the concentration of 2'Deoxyadenosine or 2'-deoxyadenosine monophosphate is of about 10 mg/L. In another embodiment, the concentration of 2'Deoxyadenosine or 2'-deoxyadenosine monophosphate is of about 20 mg/L.

Adenosine or adenosine monophosphate can be found in a range between 0.1 and 100 mg/L, preferably between 1 and 100 mg/L. In a particular embodiment, the concentration of adenosine or adenosine monophosphate is of about 5 mg/L. In another embodiment, the concentration of adenosine or adenosine monophosphate is of about 10 mg/L. In another embodiment, the concentration of adenosine or adenosine monophosphate is of about 20 mg/L.

2'-deoxycytidine or 2'-deoxycytidine monophosphate can be found in a range between 0.1 and 100 mg/L, preferably between 1 and 100 mg/L. In a particular embodiment, the concentration of 2'Deoxycytidine or of 2'-deoxycytidine monophosphate is of about 5 mg/L. In another embodiment, the concentration of 2'Deoxycytidine or of 2'-deoxycytidine monophosphate is of about 10 mg/L. In another embodiment, the concentration of 2'Deoxycytidine or of 2'-deoxycytidine monophosphate is of about 20 mg/L.

Deoxycytidine or cytidine monophosphate can be found in a range between 0.1 and 100 mg/L, preferably between 1 and 100 mg/L. In a particular embodiment, the concentration of cytidine or of cytidine monophosphate is of about 5 mg/L. In another embodiment, the concentration of cytidine or of cytidine monophosphate is of about 10 mg/L. In another embodiment, the concentration of cytidine or of ycytidine monophosphate is of about 20 mg/L.

2'-deoxyguanosine or 2'-deoxyguanosine monophosphate can be found in a range between 0.1 and 100 mg/L, preferably between 1 and 100 mg/L. In a particular embodiment, the concentration of 2'Deoxyguanosine or 2'-deoxyguanosine monophosphate is of about 5 mg/L. In another embodiment, the concentration of 2'Deoxyguanosine or 2'-deoxyguanosine monophosphate is of about 10 mg/L. In another embodiment, the concentration of 2'Deoxyguanosine or 2'-deoxyguanosine monophosphate is of about 20 mg/L.

Guanosine or guanosine monophosphate can be found in a range between 0.1 and 100 mg/L, preferably between 1 and 100 mg/L. In a particular embodiment, the concentration of guanosine or guanosine monophosphate is of about 5 mg/L. In another embodiment, the concentration of guanosine or guanosine monophosphate is of about 10 mg/L. In another embodiment, the concentration of guanosine or guanosine monophosphate is of about 20 mg/L.

When present, 5-Methyl-deoxycytidine or 5-Methyl-deoxycytidine monophosphate can be found in a range between 0.01 and 100 mg/L, preferably between 0.1 and 10 mg/L. In a particular embodiment, the concentration of 5-Methyl-deoxycytidine or 5-Methyl-deoxycytidine monophosphate is of about 0.05 mg/L. In another embodiment, the concentration of 5-Methyl-deoxycytidine or 5-Methyl-deoxycytidine monophosphate is of about 0.1 mg/L. In another embodiment, the concentration of 5-Methyl-deoxycytidine or 5-Methyl-deoxycytidine monophosphate is of about 0.2 mg/L.

Uridine or uridine monophosphate can be found in a range of between 0.01 to 100 mg/L, preferably between 0.1 and 10 mg/L. In a particular embodiment, the concentration of uridine or uridine monophosphate is of about 0.5 mg/L. In another embodiment, the concentration of uridine or uridine monophosphate is of about 1 mg/L. In another embodiment, the concentration of uridine or uridine monophosphate is of about 2 mg/L.

Thymidine or thymidine monophosphate can be found at a a concentration range between 0.01 to 100 mg/L, preferably between 0.1 to 50 mg/L. In a particular embodiment, the concentration of thymidine or thymidine monophosphate is about 5 mg/L. In another embodiment, the concentration of thymidine or thymidine monophosphate is about 11 mg/L. In another embodiment, the concentration of thymidine or thymidine monophosphate is about 20 mg/L.

The serum-free culture medium of the invention comprises a mixture of enzymatic cofactors. A used herein, the term "enzymatic cofactor" or "enzyme cofactor" refers to a compound of non-proteinaceous structure which is required by an enzyme to perform its catalytic activity. Cofactors are classified into two groups: (a) metals or metalloorganic compounds; and (b) organic molecules, or coenzymes. Coenzymes can be further divided into two subgroups. In the first of these, the coenzyme is attached to the active site and can be separated, usually reversibly, from it. Thiamine pyrophosphate and pyridoxal phosphate are good examples of such coenzymes. The coenzymes of the second group are not parts of the active site, but are specific and necessary reagents of the catalyzed reactions. Suitable enzymatic cofactors for the serum-free culture medium of the invention include, without limitation NAD, NADP, NADH, NADPH, FAD and FMN.

In another embodiment, mixture of enzymatic cofactors of the serum-free medium of the invention comprises co-carboxylase, Coenzyme A, diphosphopyridine nucleotide (NAD) and flavin adenine dinucleotide (FAD) and triphosphopyridine Nucleotide (NADP). In one embodiment, the mixture of enzymatic cofactors contains NAD but lacks NADP.

The term co-carboxylase, also known as thiamine pyrophosphate co-carboxylase as used herein refers to the coenzyme form of Vitamin B1 present in many animal tissues. It is a required intermediate in the pyruvate dehydrogenase complex and the ketoglutarate dehydrogenase complex. Is has the molecular formula C₁₂H₁₉ClN₄O₇P₂S. The concentration of co-carboxylase within the serum-free culture medium of the invention is in a range between 0.1 to 100 mg/L, more preferably between 1 to 10 mg/L. In a particular embodiment, the concentration of co-carboxylase in the culture medium of the invention is of about 0.5 mg/L. In another embodiment, the concentration of co-carboxylase in the culture medium of the invention is of about 1 mg/L. In another embodiment, the concentration of co-carboxylase in the culture medium of the invention is of about 2 mg/L.

The term Coenzyme A, as used herein, also known as S-Acetyl coenzyme A refers to acyl-CoA having acetyl as its S-acetyl component. It has a role as an effector, a coenzyme, an acyl donor and a fundamental metabolite. It derives from an acetic acid and a coenzyme A. It is a conjugate acid of an acetyl-CoA. The concentration of coenzyme A within the serum-free culture medium of the invention is in a range between 0.1 to 100 mg/L, more preferably between 1 to 10 mg/L. In a particular embodiment, the concentration of coenzyme A in the culture medium of the invention is of about 1.25 mg/L. In another embodiment, the concentration of coenzyme A in the culture medium of the invention is of about 2.5 mg/L. In another embodiment, the concentration of coenzyme A in the culture medium of the invention is of about 5 mg/L.

The term "diphosphopyridine nucleotide" (NAD), as used herein, also known as alpha-NAD, makes reference to a cofactor that is central to metabolism. Found in all living cells, NAD is called a dinucleotide because it consists of two nucleotides joined through their phosphate groups. One nucleotide contains an adenine nucleobase and the other nicotinamide. NAD exists in two forms: an oxidized and reduced form, abbreviated as NAD+ and NADH respectively. It has the molecular formula C₂₁H₂₇N₇O₁₄P₂. The concentration of NAD within the serum-free culture medium of the invention is in a range between 0.1 to 100 mg/L, more preferably between 1 to 20 mg/L. In a particular embodiment, the concentration of NAD in the culture medium of the invention is of about 3.5 mg/L. In another embodiment, the concentration of NAD in the culture medium of the invention is of about 7 mg/L. In another embodiment, the concentration of NAD in the culture medium of the invention is of about 14 mg/L.

The term flavin adenine dinucleotide (FAD), also known as flavin-adenine dinucleotide or flavitan, refers to a flavin adenine dinucleotide in which the substituent at position 10 of the flavin nucleus is a 5'-adenosyldiphosphoribityl group. It has a role as a human metabolite, an Escherichia coli metabolite, a mouse metabolite, a prosthetic group and a cofactor. It has the molecular formula C₂₇H₃₃N₉O₁₅P₂. The concentration of FAD within the serum-free culture medium of the invention is in a range between 0.1 to 100 mg/L, more preferably between 1 to 10 mg/L. In a particular embodiment, the concentration of FAD in the culture medium of the invention is of about 0.5 mg/L. In another embodiment, the concentration of FAD in the culture medium of the invention is of about 1 mg/L. In another embodiment, the concentration of FAD in the culture medium of the invention is of about 2 mg/L.

As used herein, the term triphosphopyridine Nucleotide (NADP), also known as codehydrogenase II, or nicotinamide adenine dinucleotide phosphate refers to a coenzyme composed of ribosylnicotinamide 5'-phosphate (NMN) coupled by pyrophosphate linkage to the 5'-phosphate adenosine 2',5'-bisphosphate. It serves as an electron carrier in a number of reactions, being alternately oxidized (NADP+) and reduced (NADPH). The concentration of NADP in the culture medium of the invention is in a range between 0.01 to 100 mg/L, more particularly between 0.1 to 10 mg/L, preferably of about 1 mg/L. In a particular embodiment the concentration of NADP within the serum-free culture medium is of about 0.5 mg/L. In another embodiment the concentration of NADP in the culture medium of the invention is of about 1 mg/L. In another embodiment, the concentration of NADP in the culture medium of the invention if of about 2 mg/L.

The serum-free culture medium of the invention comprises a proteolytic digest of a microorganism or a plant or animal tissue. The term "proteolytic digest" refers to the product derived from the breakdown of proteins into smaller polypeptides or amino acids. Proteolysis is typically catalysed by cellular enzymes called proteases, but may also occur by intra-molecular digestion or different processes known by a skilled in the art. As mentioned above, the proteolytic digest will provide amino acids to the medium in the form of peptides. In a particular embodiment, the peptides are derived from the proteolytic digest of a microorganism or of a plant or animal tissue, in which case the peptide may be selected from peptone, PPLO and yeastolate.

It will be understood that, although not specifically excluded from the invention, the media defined herein usually contain a single type of proteolytic digest of a microorganism or a plant or animal tissue. Thus, in some embodiments, the media contains PPLO but lacks yeastolate. In other embodiments, the media contains yeastolate but lacks PPLO.

The term PPLO, refers to a beef heart infusion, pancreatic digest of casein and beef extract broth. In a particular embodiment, the culture medium of the invention comprises PPLO in a concentration in a range between 0.01 to 10 mg/ml, more particularly between 0.1 to 10 mg/ml. In preferred embodiment, the concentration of PPLO is of about 1 mg/ml. In a preferred embodiment, the concentration of PPLO in the serum-free culture medium of the invention is of 15 mg/ml.

The term "yeastolate" refers to are animal-free and water-soluble portions of autolyzed yeast or *Saccharomyces cerevisiae.* It comprises a mixture of peptides, amino acids, carbohydrates, simple and complex as well as vitamins. In a particular embodiment, the culture medium of the invention comprises yeastolate in a concentration in a range between 0.1 to 100 mg/ml, more particularly between 1 to 50 mg/ml. In preferred embodiment, the concentration of yeastolate is of about 10 mg/ml. In a preferred embodiment, the concentration of yeastolate in the serum-free culture medium of the invention is of 10 mg/ml. In preferred embodiment, the concentration of yeastolate is of about 7,5 mg/ml. In a preferred embodiment, the concentration of yeastolate in the serum-free culture medium of the invention is of 7,5 mg/ml.

In another embodiment, the culture medium according to the invention contains a vehicle to solubilize and deliver the lipids.

The term "vehicle for solubilizing and delivering lipids" as used herein, refers to any compound which is capable of forming a complex with lipids and of shielding their hydrophobic parts such as the complexes can be found dissolved or dispersed in an aqueous solution.

In another embodiment, the vehicle to solubilize and deliver the lipids forming part of the culture medium according to the invention is not a cyclodextrin.

As used herein, cyclodextrins refer to a macrocyclic ring of glucose subunits joined by α-1,4 glycosidic bonds and which consist of 5 or more α-D-glucopyranoside units linked 1->4, as in amylose (a fragment of starch). Typical cyclodextrins contain a number of glucose monomers ranging from six to eight units in a ring, creating a cone shape and include α-cydodextrin (6 glucose subunits), β-cydodextrin: (7 glucose subunits) and γ-cyclodextrin (8 glucose subunits). Suitable cyclodextrins for use in the present invention include any of the cyclodextrins commonly used in drug delivery such as those described by Chordiya and Senthilkumaran (2012) Research and Reviews: Journal of Pharmacy and Pharmaceutical Sciences I(I):19-29 (especially Table 2 thereof as it describes different cyclodextrins in use as carriers). These include α-cyclodextrin, β-cydodextrin, and γ-cydodextrin, or derivatives of any of these cyclodextrins. The cyclodextrin derivatives herein refers to, for example, compounds obtained by replacing hydrogen or hydroxyl group(s) of cyclodextrin with other substituent(s). Examples of such substituents include, but are not limited to, alkyl having 1 to 4 carbon atoms, alkenyl having 1 to 4 carbon atoms, and groups having a C-O bond, such as acetyl.

In another embodiment, the vehicle to solubilize and deliver the lipids forming part of the culture medium according to the invention comprises polyvinyl alcohol. PVA is well known in the art and can be commercially purchased. PVA analogues or derivatives can also be used for the preparation of the culture medium according to the present invention but PVA as disclosed herein is preferred. The skilled person knows how to prepare and dissolve PVA to obtain a suitable PVA liquid form.

In some embodiments, PVA is present in the culture medium at a concentration of at least about 0,5%, at least about 1% or at least about 2%.

In another embodiment, the vehicle to solubilize and deliver the lipids forming part of the culture medium according to the invention comprises liposomes.

The term liposomes, as used herein, refers to a closed structure comprising an outer lipid bi- or multi-layer membrane surrounding an internal aqueous space. Liposomes may be multi-laminar or unilaminar. The liposome is contemplated to range in size from 5 to 10 micro M in diameter to nanoparticle size. In certain embodiments, the liposome nanoparticle is from about 50 to 500 nm, from about 100 nm to 300 nm or from about 100 to 200 nm in diameter.

In one embodiment, the liposomes are provided at a concentration of about present 0.1% (w/w). In some embodiments, the liposomes are provided at concentrations of between 0,01 and 1%, between 0,02 and 0,9%, between 0,03 and 0,7%, between 0,04% and 0,6%, between 0,05% and 0,5%, between 0,06 and 0,4%, between 0,07 and 0,8% or between 0,09 and 0,11%.

In various embodiments, the liposomes comprise lipids, fatty acids, sterols and/or free fatty acids. In various embodiments, the liposomes comprise cholesterol and phosphatidylcholine at concentrations that provide a more physiologically relevant milieu for cell growth. In one embodiment, the liposomes contain L-alpha-phosphatidylcholine and cholesterol. In additional embodiments, the liposomes contain about 18,8 mg/ml of L-alpha-phosphatidylcholine and about 4,2 mg/ml of cholesterol. IN additional embodiments, the ratio of phosphatidylcholine and cholesterol in the liposomes is of about 4:1 (w/w).

It will be understood that, although not specifically excluded from the invention, the media defined herein usually contain a single type of vehicle for solubilizing and delivering lipids. Thus, in some embodiments, the medium according to the present invention contains a lipid-binding protein but lacks cyclodextrins, polyvinyl alcohol (PVA) and liposomes. In some embodiments, the medium according to the present invention contains a polyvinyl alcohol (PVA) but lacks a lipid-binding protein, cyclodextrin and liposomes. In some embodiments, the medium according to the present invention contains a liposomes, but lacks lipid-binding protein, polyvinyl alcohol (PVA) or a cyclodextrin.

In another embodiment, the serum-free culture medium of the invention also comprises a polyamine. The term "polyamine" refers to any of a group of organic compounds composed of carbon, nitrogen, and hydrogen, and containing two or more amino groups. For example, the term encompasses molecules selected from the group consisting of cadaverine, putrescine, spermidine, spermine, agmatine, and ornithine.

In a preferred embodiment, the polyamine is spermine. The term spermine, also known as musculamine, or neuridine refers to a spermidine-derived biogenic polyamine found as a polycation at all pH values. The concentration of spermine in the culture medium of the invention is in a range between 0.01 to 100 mg/L, more particularly between 0.1 to 50 mg/L, preferably of about 10 mg/L. In a preferred embodiment the concentration of spermine is of about 1 mg/ml.

In a particular embodiment, the medium of the invention further comprises one or more organic acids or a salt thereof.

As used herein "organic salts" are defined as compounds build on a carbon skeleton, containing a functional group with acidic properties, usually weaker than mineral acid. This term includes in particular carboxylic acids and sulfonic acids, containing the group -SO₂OH. Carboxylic acids are characterized with the presence of a carboxyl group (-COOH) composed of two functional groups: a hydroxyl group (-OH) that is bonded to a carbonyl group (C=0). Organic acids are written in a condensed form R-COOH. Carboxylic acids include aliphatic, aromatic and cycloaliphatic carboxylic acids, depending on the structure of the carbon skeleton (R).

Suitable organic acids to be used in the culture medium according to the invention include, without limitation, one or more of lactic acid, acetic acid, glucuronic acid, succinic acid, propionic acid, butyric acid, methyl butyric acid, hydroxybutyric acid, aminobutyric acid (in particular GABA, gamma-aminobutyric acid), valeric acid, formic acid, aspartic acid, fumaric acid, oxalic acid, orotic acid, ketoglutaric acids, citric acid, glutamic acid, glyoxylic acid, glycolic acid, pyruvic acid, malic acid, sorbic acid and tartaric acid.

In another embodiment, the organic acids present in the culture medium of the invention are acetate or glucuronate. In additional embodiments, acetate is found in the culture medium at concentrations of at least about 41,5 mg/L, at least about 83 mg/L or at least about 166 mg/L. In other embodiments, acetate is found in the culture medium at concentrations of between 1 and 500 mg/L, between 10 and 400 mg/L, between 20 and 300 mg/L or between 40 and 200 mg/L.

In additional embodiments, glucuronate is found in the culture medium at concentrations of about 8,40 mg/L, about 4,2 mg/L or about 2.1 mg/L. In additional embodiments, acetate is found in the culture medium at concentrations of between about 0,1 mg/L and 50 mg/L, of between 0,2 and 40 mg/L, of between 0,5 and 30 mg/L, of between 1 and 20 mg/L or of between 2 and 10 mg/L.

In another embodiment, the serum-free media of the invention lacks RNA, including RNA from any source such as RNA from yeast.

In a particular embodiment the serum-free culture medium of the invention further comprises an antioxidant.

As used herein, an "antioxidant" can be defined as "any substance that delays, prevents or removes oxidative damage to a target molecule". A target molecule can be any oxidizable substrate, e.g., any organic molecule found in vivo. In some embodiments, an antioxidant is a substance that significantly delays or prevents oxidation of an oxidizable substrate. In some embodiments, an antioxidant is capable of significantly delaying or preventing oxidation of an oxidizable substrate even when present at low concentrations compared with those of an oxidizable substrate

As used herein, the term "antioxidant" includes compounds with a known structure as well as compositions that may be at least partially uncharacterized. An antioxidant can be a naturally occurring compound or may be a compound invented by man. Exemplary antioxidants include vitamin C, vitamin E, lipoic acid, L-sulforaphane, reduced L-glutathione, butylated hydroxyanisole, alpha tocopherol, deferoxamine, resveratrol, N-acetylcysteine, Trolox, curcumin, morin hydrate, vitamin A, a vitamin B (e.g., vitamin B I, B2, B6, and/or B12), coenzyme Q, green tea (epigallocatechin gallate-EGCG), citric acid, oxalic acid, phytic acid, chicoric acid, chlorogenic acid, cinnamic acid, ellagic acid, gallic acid, gallotannins, rosmarinic acid, silymarin, eugenol, manganese, zinc, adenosine, transferrin, lactoferrin, cysteine, histidine-containing dipeptides, pyridoxamine, carotenoids, flavonoids (e.g., kaempferol, myricetin, silymarin, quercetin), flavones and flavonols (e.g., planar flavones and flavonols with a 7-hydroxyl group), other phenolic compounds (e.g., plant-derived phenolics), melatonin, coelenterazine, nordihydroguaiaretic acid (NDGA), curcumin (diferuloylmethane), 21 -amino steroids (also called lazaroids), various antibiotics (e.g., tetracyclines), azoles (e.g., ketoconazole), thiols (e.g., mercaptopropionylglycine), metal ion chelators (e.g., iron chelators such as ICRF-187, deferasirox, deferiprone, hydroxypyridones), fullerenes, xanthine oxidase inhibitors (e.g., purine analogues such as allopurinol, oxypurinol, and tisopurine and other compounds such as febuxostat and inositols), selenium (e.g., sodium selenite, selenous acid), uric acid, bilirubin, trehalose, lipid-soluble chain-breaking antioxidants such as BHT and ethoxyquin, superoxide dismutase (SOD), catalase, superoxide dismutase (SOD).

In a preferred embodiment the antioxidant is reduced glutathione. The concentration of glutathione in the medium is of at least 20 mg/L, of at least 10 mg/L or of at least 5 mg/L. In other embodiments, reduced glutathione is present in the culture medium at a concentration of between 1 and 50 mg/L, between 2 and 40 mg/L, between 3 and 30 mg/L, between 4 and 20 g/L or between 5 and 10 mg/L.

In another embodiment, the serum-free culture medium of the invention further comprises one or more detergents. Detergents are amphipathic molecules with a polar portion and a hydrophobic portion. Detergents respond to an aqueous environment following the same principles as do membrane lipids. Suitable detergents for the culture medium of the invention include without limitation

In a particular embodiment, the detergent is selected from Tween 40 and/or Tween 80. In a particular embodiment, the detergent is Tween 80, in which case, the concentration is of at least 0.00075% (w/w). In another embodiment the detergent present in the culture medium of the invention is Tween 40, in which case, the concentration is of at least 0.0015%. In another embodiment the detergent is Tween 80, Tween 40 or a mixture thereof.

In preferred embodiments, the serum-free culture media according to the present invention comprise the vB2, vB3, vB4, vB6, vB7, vB8, vB9, vB10 or vB11 as defined in Figure 8.

In another embodiment, the serum-free culture medium of the invention comprises components defined in the "Components" column in the Table 2 at concentrations indicated in the "Concentration 1" column, at the concentrations indicated in the "Concentration 2" column or at the concentrations indicated in the "Concentration 3" column.

| **Components** | **Concentration 1 (mg/L)** | **Concentration 2 (mg/L)** | **Concentration 3 (mg/L)** |
|---|---|---|---|
| Glycine | 100.0 | 50.0 | 25.0 |
| Hydroxy L-proline | 20.0 | 10.0 | 5.0 |
| L-Alanine | 50.0 | 25.0 | 12,5 |
| L-Arginine hydrochloride | 140.0 | 70.0 | 35.0 |
| L-Aspartic acid | 60.0 | 30.0 | 15.0 |
| L-Cysteine | 399.76 | 199.88 | 99,94 |
| L-Cystine | 40.0 | 20.0 | 10.0 |
| L-Glutamic Acid | 150.0 | 75.0 | 37.5 |
| L-Histidine hydrochloride-H2O | 40.0 | 20.0 | 10.0 |
| L-Isoleucine | 40.0 | 20.0 | 10.0 |
| L-Leucine | 120.0 | 60.0 | 30.0 |
| L-Lysine hydrochloride | 140.0 | 70.0 | 35.0 |
| L-Methionine | 30.0 | 15.0 | 7.5 |
| L-Phenylalanine | 50.0 | 25.0 | 12.5 |
| L-Proline | 80.0 | 40.0 | 20.0 |
| L-Serine | 50.0 | 25.0 | 12.5 |
| L-Threonine | 60.0 | 30.0 | 15.0 |
| L-Tryptophan | 20.0 | 10.0 | 5.0 |
| L-Tyrosine | 80.0 | 40.0 | 20.0 |
| L-Valine | 50.0 | 25.0 | 12.5 |
| Ascorbic Acid | 100.0 | 50.0 | 25.0 |
| Biotin | 0.02 | 0.01 | 0.005 |
| Cholesterol | 0.4 | 0.2 | 0.1 |
| Choline chloride | 1.0 | 0.5 | 0.25 |
| D-Calcium pantothenate | 0.02 | 0.01 | 0.005 |
| Folic Acid | 0.02 | 0.01 | 0.005 |
| Niacinamide | 0.05 | 0.025 | 0.0125 |
| Nicotinic acid (Niacin) | 0.05 | 0.025 | 0.0125 |
| Para-Aminobenzoic Acid | 0.10 | 0.05 | 0.025 |
| Pyridoxal hydrochloride | 0.05 | 0.025 | 0.0125 |
| Pyridoxine hydrochloride | 0.05 | 0.025 | 0.0125 |
| Riboflavin | 0.02 | 0.01 | 0.005 |
| Thiamine hydrochloride | 0.02 | 0.01 | 0.005 |
| i-Inositol | 0.1 | 0.05 | 0.0025 |
| Calcium Chloride (CaCl2-2H2O) | 528.0 | 264.0 | 132.0 |
| Magnesium Sulfate (MgSO4-7H2O) | 400.0 | 200.0 | 100.0 |
| Potassium Chloride (KCl) | 800.0 | 400.0 | 200.0 |
| Sodium Bicarbonate (NaHCO3) | 4400.0 | 2200.0 | 1100.0 |
| Sodium Chloride (NaCl) Sodium Phosphate monobasic (NaH2PO4- | 13598.0 | 6799.0 | 3399.5 75.4 |
| 2H2O) | 316.0 | 158.0 | |
| 2'Deoxyadenosine | 20.0 | 10.0 | 5.0 |
| 2'Deoxycytidine | 20.0 | 10.0 | 5.0 |
| 2'Deoxyguanosine | 20.0 | 10.0 | 5.0 |
| 5-Methyl-deoxycytidine | 0.2 | 0.1 | 0.05 |
| Co-carboxylase | 2.0 | 1.0 | 0.05 |
| Coenzyme A | 5.0 | 2.5 | 1.25 |
| D-Glucose (Dextrose) | 2000.0 | 1000.0 | 500.0 |
| Diphosphopyridine nucleotide (NAD) | 14.0 | 7.0 | 3.5 |
| FAD (flavin adenine dinucleotide) | 2.0 | 1.0 | 0.05 |
| Glutathione (reduced) | 20.0 | 10.0 | 5.0 |
| Phenol Red | 40.0 | 20.0 | 10.0 |
| Sodium acetate-3H2O | 166.0 | 83.0 | 41.5 |
| Sodium glucuronate-H2O | 8.40 | 4.2 | 2.1 |
| Thymidine | 20.0 | 10.0 | 5.0 |
| Triphosphopyridine Nucleotide (NADP) | 2.0 | 1.0 | 0.5 |
| Tween 80® | 10.0 | 5.0 | 2.5 |
| Uridine 5'- triphosphate | 2.0 | 1.0 | 0.5 |
| Table 2: Composition of the CMRL medium at 1x, 2x or 0,5 x. | | | |

In another embodiment, the medium according to the present invention further comprises a pH indicator.

Suitable pH indicators for use in the culture media according to the invention are those pH indicators with a transition range that extends below pH 7.0. Non-limiting examples of suitable pH indicators include halochromic compounds with a transition pH range that extends below the pH of the inoculated culture medium. A suitable pH indicator will have a transition pH range that extends far enough below the pH of the inoculated medium to detect (visually and/or by using an imaging system) a change in the pH indicator in or adjacent to a growing colony of acid-producing bacteria. Preferably, the pH indicator will have a transition pH range with a low endpoint that is not less than 0.25 pH units below the pH of the inoculated culture medium. More preferably, the pH indicator will have a transition pH range with a low endpoint that is not less than 0.5 pH units below the pH of the inoculated culture medium. Even more preferably, the pH indicator will have a transition pH range that extends not less than 1.0 pH unit below the pH of the inoculated culture medium. Most preferably, the pH indicator will have a transition pH range with a low endpoint that is about 3.5. Nonlimiting examples of suitable pH indicators include bromcresol purple, bromphenol blue, chlorophenol red, and bromcresol green.

A pH indicator such as phenol red may be added that turns yellow in acidic conditions (<6.8), and pink in basic conditions (>8.2). Incubation pH is controlled by the high CO₂ levels in the incubator of 5% or more. In a particular embodiment, the serum-free medium of the invention comprises phenol red pH 7 at a concentration in a range between 0.00001% to 0.1%, more preferably between 0.0001% to 0.01%, preferably at about 0.0005%, preferably at about 0.0035%.

In another embodiment, the medium according to the present invention further comprises an antibiotic.

The term "antibiotic" is intended to mean any compound capable of preventing or slowing down the growth of a bacterium. Suitable antibiotics for use in the present invention includes antibiotics of the aminoglycoside family, of the ansamycin family, of the carbacephem family, of the cephalsporine family, of the macrolide family, of the monobactam family, of the penicillin family, of the Sulfonamide family or of the tetracycline family.

An aminoglycoside, including, but not limited to amikacin, gentamicin, kanamycin, neomycin, netilmicin, streptomycin, tobramycin, and paromomycin; an ansamycin, including, but not limited to, geldanamycin and herbimycin. A carbacephem, including, but not limited to, loracarbef. A carbapenem, including, but not limited to, ertapenem, doripenem, imipenem/cilastatin, and meropenem. First generation cephalosporins, including, but not limited to, cefadroxil, cefazolin, cefalotin, and cephalexin. Second generation cephalosporins, including, but not limited to, cefaclor, cefamandole, cefoxitin, cefprozil, and cefuroxime. Third generation cephalosporins, including, but not limited to, cefixime, cefdinir, cefditoren, cefoperazone, cefotaxime, cefpodoxime, ceftazidime, ceftibuten, ceftizoxime, ceftriaxone, and cefdinir. Fourth generation cephalosporins, including, but not limited to cefepime. Glycopeptides, including, but not limited to, teicoplanin and vancomycin. Macrolides, including, but not limited to azithromycin, clarithromycin, dirithromycin, erythromycin, roxithromycin, telithromycin, and spectinomycin. Monobactams, including but not limited to aztreonam. Penicillins, including, but not limited to, amoxicillin, ampicillin, azlocillin, carbenicillin, cloxacillin, dicloxacillin, flucloxacillin, mezlocillin, nafcillin, penicillin, piperacillin, and ticarcillin. Polypeptides, including, but not limited to, bacitracin, colistin, and polymyxin B. Quinolones, including, but not limited to, ciprofloxacin, enoxacin, gatifloxacin, levofloxacin, lomefloxacin, moxifloxacin, norfloxacin, ofloxacin, and trovafloxacin. Sulfonamides, including, but not limited to, mafenide, prontosil, sulfacetamide, sulfamethizole, sulfanilamide, sulfasalazine, sulfisoxazole, trimethoprim, and trimethoprim-sulfamethoxazole (co-trimoxazole (TMP-SMX). Tetracyclines, including, but not limited to demeclocycline, doxycycline, minocycline, oxytetracycline, and tetracycline. And other antibiotics, including, but not limited to, arsphenamine, chloramphenicol, clindamycin, lincomycin, ethambutol, fosfomycin, fusidic acid, furazolidone, isoniazid, linezolid, metronidazole, mupirocin, nitrofurantoin, platensimycin, pyrazinamide, quinupristin/dalfopristin, rifampin, and tinidazole.

The serum-free culture medium of the invention preferably lacks a chemical buffering system or a buffering agent. The terms "buffer," "buffering system," and/or "buffer solution" refer to a solution, which reduces the change of pH upon addition of small amounts of acid or base, or upon dilution. The term "buffering agent" refers to a weak acid or weak base in a buffer solution.

### Methods for the culture of Mycoplasma

In another aspect, the invention relates to a method for the culture of a Mycoplasma strain comprising placing an inoculum of said strain into the media according to the invention and maintaining the inoculated media under conditions adequate for the proliferation of the Mycoplasma strain.

In another aspect, the invention relates to a method for obtaining a biomass of a Mycoplasma strain which comprises placing an inoculum of said Mycoplasma strain into the media according to the invention and maintaining the inoculated media under conditions adequate for the proliferation of the Mycoplasma strain and the formation of the biomass.

In one embodiment, the Mycoplasma is *Mycoplasma hyopneumoniae.* In another embodiment, he Mycoplasma is a *Mycoplasma hyopenumoniae* wild-type strain or a Mycoplasma pneumoniae variant strain. In yet another embodiment, the inoculum of *Mycoplasma hyopneumoniae* cells contains about 10⁷ cells/ml.

In some embodiments, the serum-free media can be used in methods for culturing cells.. In some embodiments, cells are cultured for at least at or about 1, 2, 3, 4, 5, 6, 7, 8, 9, 10, 11, 12, 13, 14, 15, 16, 17, 18, 19, or 20 days with the provided serum- free media. In some embodiments, cells are cultured for about 2-3 days, 3-4 days, 4-5 days, 5-6 days, 6-7 days, 7-8 days, 8-9 days, 9-10 days, 10-11 days, 11-12 days, 12-13 days, 13-14 days, 14-15 days, 15-16 days, 16-17 days, 17-18 days, 18-19 days, or 19-20 days, each inclusive. In some embodiments, the cells are cultured at about 37 degrees centigrade In some embodiments, the cells are cultured in 5 percent C0₂/95 percent air atmosphere. In some embodiments, the culturing can be carried for more than or more than about 1, 2, 3, 4, 5, 6, 7, 8, 9, 10, 11, 12, 13, 14, 15, 16, 17, 18, 19, or 20 days without changing the media when culturing cells. In some embodiments, methods of perfusion, such as semi-continuous perfusion, can be employed in connection with culturing the cells.

In some embodiments, provided herein are methods for culturing cells, such as for cultivation, expansion or proliferation of cells. In some embodiments, the methods are carried out to expand the cells at least at or about 1.5-fold, 2-fold, 3-fold, 4-fold, 5-fold, 6-fold, 7-fold, 8-fold, 9-fold, 10-fold, 15-fold, 20-fold, 25-fold, 30-fold, 35-fold, 40-fold, 45-fold, 50-fold, 55- fold, 60-fold, 65-fold, 70-fold, 75-fold, 80-fold, 85-fold, 90-fold, 95-fold, 100-fold or more, such as following culture in the presence of serum-free media for at or about 1, 2, 3, 4, 5, 6, 7, 8, 9, 10, 11, 12, 13, 14, 15, 16, 17, 18, 19, 20 or more days. In some embodiments, the cells expand at least about 5-fold, 10-fold, 20-fold, 30-fold, 40-fold, or 50-fold or more after culture in the serum-free medium formulation for about 5 to 6 days. In some embodiments, the expansion is comparable or improved compared to culture with serum-containing media under the same conditions, such as following culture for at or about 1, 2, 3, 4, 5, 6, 7, 8, 9, 10, 11, 12, 13, 14, 15, 16, 17, 18, 19, 20 or more days.

The invention is defined by the following aspects:
1. A serum-free culture medium suitable for the culture of *Mycoplasma hyopneumoniae* that comprises:
(i) a carbon source comprising pyruvate,
(ii) an amino acid mixture,
(iii) a vitamin mixture,
(iv) lipids,
(v) inorganic salts,
(vi) a nucleoside mixture comprising thymidine,
(vii) a mixture of enzymatic cofactors,
(viii) a pyruvate salt,
(ix) a proteolytic digest of a microorganism or of a plant or animal tissue and
(x) a vehicle to solubilize and deliver the lipids.
2. The medium according to any preceding aspect wherein the pyruvate salt is sodium pyruvate.
3. The medium according to aspect 23 wherein the sodium pyruvate is found at a concentration of at least 2.2 g/L, preferably at least 4 g/L.
4. The medium of any preceding aspect wherein the carbon source comprises glucose, acetate, glucuronate or a combination thereof.
5. The medium of any preceding aspect further comprising glycerol at a concentration of at least 0,01%.
6. The medium according to any of aspects 1 to 3 wherein the amino acids include Glycine, Hydroxy L-proline, L-Alanine, L-Arginine, L-Aspartic acid, L-Cysteine, L-Cystine, L-Glutamic Acid, L-Histidine, L-Isoleucine, L-Leucine, L-Lysine, L-Methionine, L-Phenylalanine, L-Proline, L-Serine, L-Threonine, L-Tryptophan, L-Tyrosine, L-Valine and L-glutamine.
7. The medium according to aspect 6 wherein the amino acids further incude L-asparfic acid and L-glutamic acid.
8. The medium of aspect 6 wherein the content in L-Glutamine is of least 2 mM.
9. The medium according to any preceding aspect wherein the vitamins include ascorbic acid, biotin, D-Calcium pantothenate, folic acid, niacinamide, nicotinic acid (Niacin), para-aminobenzoic acid, pyridoxal, pyridoxine, riboflavin, thiamine, i-Inositol, and choline, thioctic acid, pyridoxamine, or any precursor thereof.
10. The medium according to aspect 9 wherein pyridoxamine is found at a concentration of at least 0,5 µg/mL, nicotinic acid is found at a concentration of at least 0,5 µg/mL and/or riboflavin is found at a concentration of at least 0,5 µg/mL.
11. The medium according to aspect 10 wherein the concentration of nicotinic acid is of 0,025 µg/mL or less and/or wherein the concentration of riboflavin is of 0.01 µg/mL or less.
12. The medium according to any preceding aspect wherein the content of thioctic acid is of at least 0,2 µg/ml.
13. The medium according to any preceding aspect wherein the lipids are cholesterol, sphingomyelin, phosphatidylcholine, L-α-Phosphatidyl-DL-glycerol, 1,2-Dipalmitoyl-sn-glycero-3-phosphocholine or a combination thereof.
14. The medium according to aspect 13 wherein the cholesterol is present at a concentration of about 30 µg/mL.
15. The medium according to aspects 13 or 14 wherein the sphingomyelin is present at a concentration of less than about 10 µg/mL.
16. The medium according to any of aspects 13 to 14 wherein the phosphatidylcoholine is present at a concentration of ca. 40 µg/mL, preferably at ca. 60 µg/mL.
17. The medium according to any of aspects 13 to 16 wherein the L-α-Phosphatidyl-DL-glycerol is present at a concentration of between. 10 and 20 µg/mL.
18. The medium according to any of aspects 13 to 17 wherein the 1,2-Dipalmitoyl-sn-glycero-3-phosphocholine is present at a concentration of 10 to. 20 µg/mL.
19. The medium according to any preceding aspect wherein the inorganic salts include at least one salt wherein the cation is calcium, magnesium, potassium or sodium and wherein the anion is chloride, sulfate, bicarbonate or phosphate monobasic.
20. The medium according to any preceding aspect wherein the nucleoside mixture further comprises one or more deoxyribonucleosides or one or more ribonucleosides.
21. The medium according to aspect 20 wherein the deoxyribonucleoside is 2'-deoxyadenosine, 2'-deoxycytidine, 2'-deoxyguanosine, 5-methyl-deoxycytidine or a mixture thereof and wherein the ribonucleosides are thymidine, uridine-5'-triphosphate, thymidine, adenosine, cytidine, guanosine, thymine or a mixture thereof.
22. The medium according to any of aspects 1 to 21 wherein the thymidine content is of at least 40 µg/mL.
23. The medium according to any preceding aspect wherein the medium lacks RNA and contains ribonucleosides.
24. The medium according to any preceding aspect wherein the enzymatic cofactor is co-carboxylase, Coenzyme A, diphosphopyridine nucleotide (NAD) and flavin adenine dinucleotide (FAD) and triphosphopyridine Nucleotide (NADP).
25. The medium of any preceding aspect wherein the proteolytic digest of a microorganism or of a plant or animal tissue is yeastolate.
26. The method of aspect 25 wherein the yeastolate is present at a concentration of about 7,5 mg/L.
27. The medium according to any preceding aspect wherein the vehicle to solubilize and deliver the lipids is selected from the group of a lipid-binding protein, PVA and a liposome.
28. The medium according to any preceding aspect wherein the vehicle to solubilize and deliver the lipids is a lipid-binding protein, preferably delipidated BSA.
29. The medium according to aspect 28 wherein the delipidated bovine serum albumin is complexed to cholesterol.,
30. The medium according to any of aspect 28 or 29 wherein the BSA is found at a concentration of 0,5% (w/w) or less, preferably at 0,3% (w/w) or less.
31. The medium according to any preceding aspect further comprising a polyamine.
32. The medium according to aspect 28 wherein the polyamine is spermine.
33. The method according to aspect 29 wherein the spermine is present at a concentration of about 10 µg/mL.
34. The medium according to any preceding aspect further comprising an antioxidant.
35. The medium according to aspect 34 wherein the antioxidant is reduced glutathione.
36. The medium according to any preceding aspect further comprising a detergent.
37. The medium according to aspect 36 wherein the detergent is Tween 80.
38. The medium according to aspect 34 wherein the concentration of Tween 80 is of at least 0.00075%.
39. The medium according to any preceding aspect which lacks oleic acid, palmitic acid or both.
40. The medium according to any preceding aspect wherein the components defined in the "component" column in the following table are present at concentrations indicated in the "Concentration" column.

| **Components** | **Concentration (mg/L)** |
|---|---|
| Glycine | 25.0 |
| Hydroxy L-proline | 5.0 |
| L-Alanine | 12,5 |
| L-Arginine hydrochloride | 35.0 |
| L-Aspartic acid | 15.0 |
| L-Cysteine | 99,94 |
| L-Cystine | 10.0 |
| L-Glutamic Acid | 37.5 |
| L-Histidine hydrochloride-H2O | 10.0 |
| L-Isoleucine | 10.0 |
| L-Leucine | 30.0 |
| L-Lysine hydrochloride | 35.0 |
| L-Methionine | 7.5 |
| L-Phenylalanine | 12.5 |
| L-Proline | 20.0 |
| L-Serine | 12.5 |
| L-Threonine | 15.0 |
| L-Tryptophan | 5.0 |
| L-Tyrosine | 20.0 |
| L-Valine | 12.5 |
| Ascorbic Acid | 25.0 |
| Biotin | 0.005 |
| Cholesterol | 0.1 |
| Choline chloride | 0.25 |
| D-Calcium pantothenate | 0.005 |
| Folic Acid | 0.005 |
| Niacinamide | 0.0125 |
| Nicotinic acid (Niacin) | 0.0125 |
| Para-Aminobenzoic Acid | 0.025 |
| Pyridoxal hydrochloride | 0.0125 |
| Pyridoxine hydrochloride | 0.0125 |
| Riboflavin | 0.005 |
| Thiamine hydrochloride | 0.005 |
| i-Inositol | 0.0025 |
| Calcium Chloride (CaCl2-2H2O) | 132.0 |
| Magnesium Sulfate (MgSO4-7H2O) | 100.0 |
| Potassium Chloride (KCl) | 200.0 |
| Sodium Bicarbonate (NaHCO3) | 1100.0 |
| Sodium Chloride (NaCl) | 3399.5 |
| Sodium Phosphate monobasic (NaH2PO4-2H2O) | 75.4 |
| 2'Deoxyadenosine | 5.0 |
| 2'Deoxycytidine | 5.0 |
| 2'Deoxyguanosine | 5.0 |
| 5-Methyl-deoxycytidine | 0.05 |
| Co-carboxylase | 0.05 |
| Coenzyme A | 1.25 |
| D-Glucose (Dextrose) | 500.0 |
| Diphosphopyridine nucleotide (NAD) | 3.5 |
| FAD (flavin adenine dinucleotide) | 0.05 |
| Glutathione (reduced) | 5.0 |
| Phenol Red | 10.0 |
| Sodium acetate-3H2O | 41.5 |
| Sodium glucuronate-H2O | 2.1 |
| Thymidine | 5.0 |
| Triphosphopyridine Nucleotide (NADP) | 0.5 |
| Tween 80® | 2.5 |
| Uridine 5'- triphosphate | 0.5 |

41. The medium according to aspect 40 wherein the content of one or more components selected from the group consisting of Tween80, thiamin, niacinamide, nicotinic acid, folic acid, pathonthenic acid, ascorbic acid, riboflavin, spermidine, Para-Aminobenzoic Acid, Pyridoxal hydrochloride, Pyridoxine hydrochloride, Histidine, Methionine, Proline, Cysteine and Tryptophan is not higher than those provided in the Table in aspect 36.
42. The medium of any preceding aspects further comprising a pH indicator, an antibiotic or a combination thereof.
43. The medum according to any preceding aspect wherein the medium lacks a buffering system.
44. The medium according to any preceding aspect wherein the medium is selected from the group consisting of the vH5, vH5.1 or vH5.2.
45. A method for the culture of a Mycoplasma strain comprising placing an inoculum of said strain into the media according to any of aspects 1 to 44 and maintaining the inoculated media under conditions adequate for the proliferation of the Mycoplasma strain.
46. A method for obtaining a biomass of a Mycoplasma strain which comprises placing an inoculum of said Mycoplasma strain into the media according to any of aspects 1 to 44 and maintaining the inoculated media under conditions adequate for the proliferation of the Mycoplasma strain and the formation of the biomass.
47. The method according to any of aspects 45 or 46 wherein the Mycoplasma is *Mycoplasma hyopenumoniae.*
48. The method according to any of aspect 47 wherein the *Mycoplasma hyopneumoniae* strain is a wild-type strain or an attenuated strain.

The invention will be described by way of the following examples which are to be considered as merely illustrative and not limitative of the scope of the invention.

### EXAMPLES

### EXAMPLE 1

### 1. First development of a serum-free medium for M. hyopneumoniae

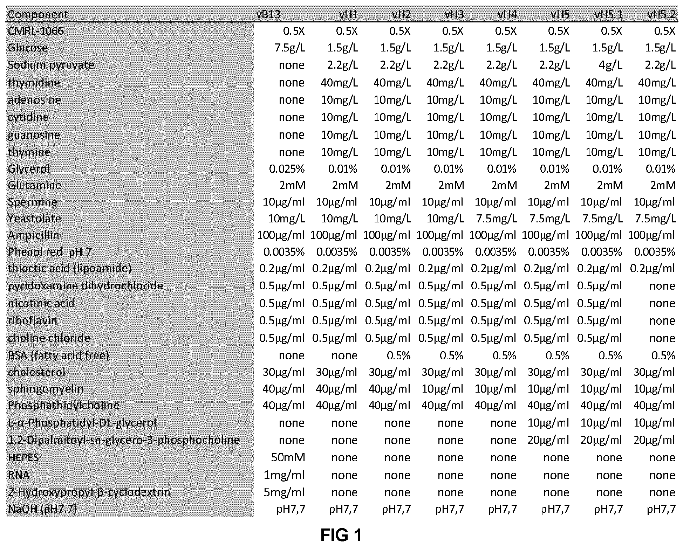

The CRG has used the *M hyopneumoniae* vaccine strain J (22JAN2016) provided by MSD and the vB13 medium as a starting point to develop a serum-free medium for *M hyopneumoniae.* The vB13 (figure 1) medium has been developed by CRG and supports robust growth of *M pneumoniae*, producing 80% of biomass when compared to rich media in a 96-well plate format, and 60-70% when the culture volume is scaled up to 5ml in 25cm2 culture flasks. In addition, the composition of this medium also tolerates serial passaging.

*M hyopneumoniae* produces less lactic acid and grows in suspension in contrast to *M pneumoniae.* This has hampered the implementation of the highthroughput method established previously to monitor growth for *M pneumoniae.* It was also found that culture conditions in a 96-well plate format were not optimal for growing *M hyopneumoniae* and that biomass estimation by protein quantification gives a high background signal for this bacterium, especially in certain media formulations. In addition, CRG has not access on a daily basis to flow cytometers to monitor growth of *M hyopneumoniae.* To overcome these difficulties the CRG has modified the screening method to monitor growth of *M. hyopneumoniae* as follows. For each experiment, 3 culture replicates of 4ml each containing the tested medium were inoculated with frozen stocks (1:200 dilutions). Frozen stocks were prepared as MSD recommended with MHM medium, which was also provided by MSD. For media comparison, the MHM rich medium was used in each experiment as the reference medium (also tested by triplicate). Cultures were incubated at 3 7°C in orbital shacking (180rpm) in 15ml conical tubes and total biomass was estimated after 4 days of culture by DNA quantification (the specific protocol can be found the methodology described below).

### Results

Using the culture conditions described above, *M. hyopneumoniae* barely grows in vB13. Hence, the vB13 version was optimized by modifying concentrations and testing the effect of new components. It was fundamental to define the precise concentration of some components when combined with others, meaning that new components were repetitively re-tested on improved versions of the medium. Following this set up, it was found that sodium pyruvate stimulated growth as previously described. Thymidine was required for growth consistent with the fact that this bacterium does not possess the thy gene. Although CMRL contains thymidine it was found that higher levels were beneficial. Also, it was found that ribonucleosides were a better option than RNA. Moreover, non-lipidated BSA complexed to cholesterol was an important growth factor together with sphingomyielin (SPM) and phosphatidylcholine (PC). Interestingly, it was found that *M hyopneumoniae* depends completely on PC and seems to be less dependent and in fact tolerates less SPM compared toM pneumoniae. Cardiolipin did not improve growth, but it was found that other phospholipids such as 1,2-Dipalmitoyl-sn-glycero-3-phosphocholine and especially L-a-Phosphatidyl-DL-glycerol improved medium performance. Addition of palmitic and oleic acid was detrimental as expected, since in contrast to *M. pneumoniae*, *M hyopneumoniae* cannot use these components to make phospholipids. On the other hand, we did not observe significant improvements when tested the effect of other components such as Tween80, Tween40, myo-inositol, thiamin, niacinamide, folic acid, pathonthenic acid, ascorbic acid, putrescine, spermidine, and several aminoacids such as Histidine, Methionine, Proline, Cysteine and Tryptophan. Some improvement adding PVA, PEG8000, or heptadecanoic acid were observed, but it was not consistent in further experiments

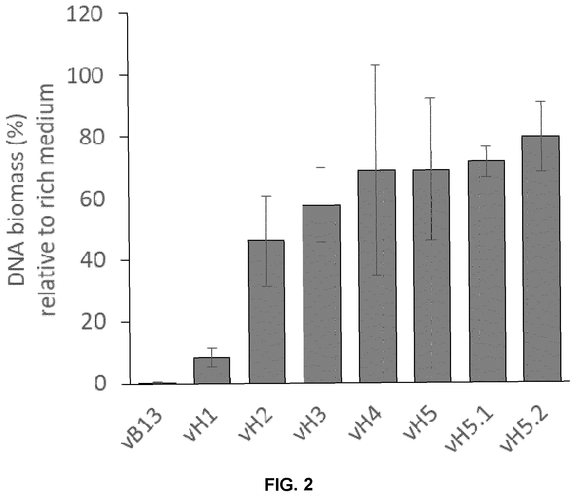

The modifications mentioned above and the refinement of the concentration of specific components have generated a serum-free version medium (vH5), which supports 57% growth of *M hyopneumoniae* compared to rich medium (Fig. 2 and Table 2. Instructions to prepare the medium are also disclosed in the methodology below).

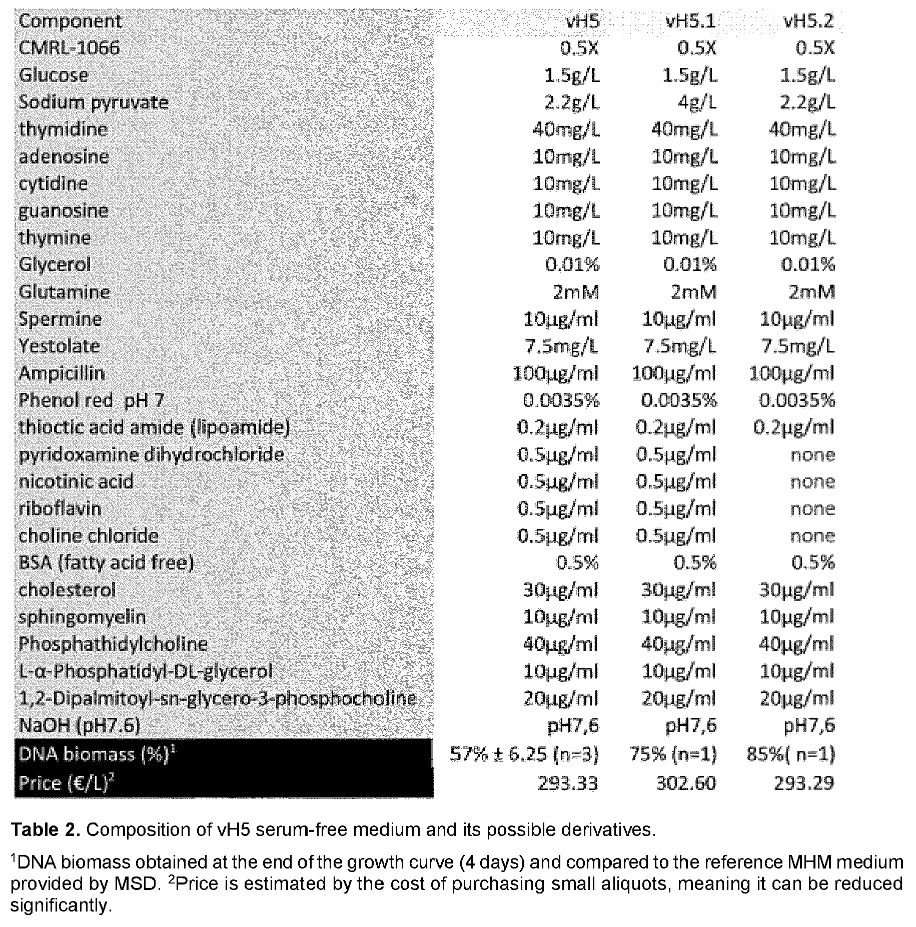

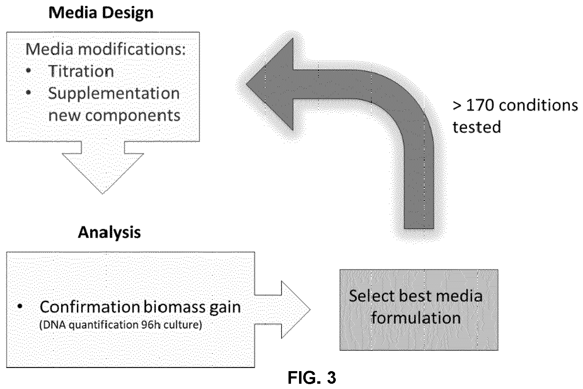

Noteworthy, it was found significant variability in the total biomass yields of both, MHM rich medium and the serum-free medium derivatives (Fig. 3). This variability hampered to precise the contribution of some components and suggests that both mediums are not robust enough; culture conditions are not optimal, and/or exists inherent variability in the method to estimate biomass.

### Summary of the experiment

It was found possible improvements to vH5 medium, including the increase of sodium pyruvate (vH5.1) or the removal of all vitamins except thioctic acid (vH5.2), which is the only vitamin not present in CMRL (Fig. 3 and Table 2). Given that these improvements arise from a single experiment (with 3 replicates), we suggest confirming these results and performing experiments to see if the combination of these changes further improves the medium performance. On the other hand, it was not possible to substitute the BSA by cyclodextrin, which resulted in a decrease in performance of 8-10% growth. Therefore, other strategies must be considered to obtain a completely animal component free medium. Finally, in order to reduce the cost of the medium, consider removing 1,2-Dipalmitoyl-sn-glycero-3-phosphocholine and especially L-a-Phosphatidyl-DL-glycerol and increasing PC to 60J.tg/ml. However, in these conditions, a 20% reduction of growth compared to vH5 medium was observed. BSA, also an expensive component, can be reduced up to 0.3% but also with concomitant reduction of growth.

### Methodology

### DNA quantification

For total DNA extraction we use the Master Pure DNA purification Kit (Epicentre, ref: MCD85201) and for DNA quantification we use a fluorometric method (QUBIT dsDNA HS assay Kit, Invitrogen, ref:Q32851). Below, there is a detailed protocol.

### Harvest and lysis of samples

- -Harvest cells by centrifugation (14100g 10min) and discard supernatant.
- (M. hyopneumoniae growth in 4ml cultures in 15ml conical tubes was normally tested. To estimate growth, the whole culture was pelleted).
- -Resuspend each pellet in 300fll Tissue and Cell Lysis solution+ 1 fll Proteinase K (Epicentre Kit).
- -incubate 15min at 65C, vortex every 5min.
- -cool sample at 37C, add 1fll RnaseA and mix thoroughly (Epicentre Kit).
- -incubate at 37C for 30min.
- -after incubation place sample on ice 5min and proceed with DNA precipitation.

### DNA precipitation

- -add 175 fll of MPC Protein precipitation reagent (Epicentre Kit) to each sample
- and vortex mix vigorously for 10 sec.
- -centrifuge for 10min at 14000rpm in a microcentrifuge.
- -transfer supernatant to a clean tube.
- -add 500 fll of isopropanol to the recovered supernatant and mix by inverting the
- tube 30-40 times.
- -incubate for 15min at RT.
- -pellet the DNA by centrifugation at 4C for 10min at 14000rpm.
- -carefully pour off the isopropanol and rinse twice the pellet with 1ml 75%
- ethanol (in each washing step repeat centrifugation).
   - remove all of the residual ethanol with a pipet and dry the pellet at RT.
   - resuspend the DNA pellet in 100 fll MilliQ water.

### DNA quantification

-measure DNA using QUBIT dsDNA HS assay Kit following manufacturer's instructions (typically we use 1:200 dilutions or more).

### Instructions for preparing 4ml of complete vH5 medium

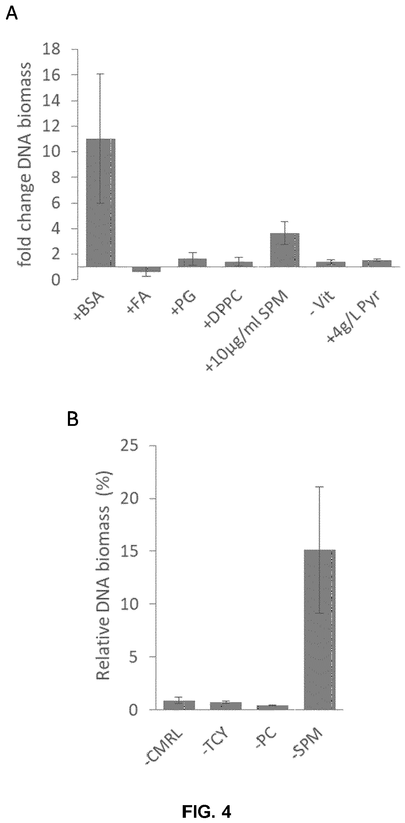

### (when preparing follow the order described in table of figure 4)

1. Prepare the base by adding the different components in the order described in the table. The base is stable at 4°C, therefore, it is useful to
   prepare a larger volume.
2. It is recommended to prepare the final medium freshly when needed by adding the remaining components in the described order to the base. Scale up the volume as desired.
3. To prepare 4 ml complete medium:
   - -Add 8uL ofPhosphathidylcholine into 2354.5ul of base and mix thoroughly.
   - -Add 2.7ul of 1,2-Dipalmitoyl-sn-glycero-3-phosphocholine and mix thoroughly.
   - -Add Sui ofL-a-Phosphatidyl-DL-glycerol and mix thoroughly.
   - -Then, add 512µl BSA+cholesterol mix and mix thoroughly (see comment 2).
   - -Finally, add Sui of Sphingomyelin and mix thoroughly.

### Comments when preparing stocks (*):

1. From a stock of 10 M NaOH (sterile solution) prepare 1M stock solution with sterile water. The media pH is adjusted at 7.4-7.6 when adding the amount indicated ofNaOH. However, the pH of the final medium can be checked and adjusted with NaOH or HCl if necessary. (Optimal pH 7.6).
2. To prepare the BSA+ cholesterol mix, homogenize by pippting up and down several times 12 ul cholesterol into 500u14% BSA. During the homogenization process you will create some foam. This step is critical to complex cholesterol to the BSA. Note, that the volumes indicated for the BSA+ cholesterol mix are for 4ml of medium but it can be scaled up to larger volumes and store aliquots at -20C. Indeed, this is recommended as we observed some variation in the performance of the medium when different BSA+ cholesterol mix were used, probably indicating different degrees of complexation.

### EXAMPLE 2

### Optimization of a serum-free medium for mycoplasma growth

### Methods and description of the invention

the development and optimization of a serum-free medium capable to support and maintain growth of *M. hyopneumoniae* is provided, In principle, the composition of the medium developed can be used as a platform to grow other related mycoplasmas species.

### 1.1 Preparation of bacterial stocks

Frozen stocks of the *M. hyopneumoniae non-pathogenic* strain J were used as starting inocula in the culture tests. Bacterial stocks were prepared as follows. A 50ml conical tube containing 10ml of modified Friss rich medium (Kamminga et al., 2017) was inoculated with 250µl of *M. hyopneumoniae* cell suspension and cultured at 37°C in an orbital shaker set at 180rpm. After 72h of growth, the 10 ml culture was diluted in 90 ml of fresh medium in an Erlenmeyer flask of 250ml. After 24h of culture, the 100 ml culture was diluted again by adding 150ml of fresh media in an Erlenmeyer flask of 500ml and cultured for 24h more. Then, bacterial cells were harvested by centrifugation (14100g, 10 min) and the pellet resuspended in 30 ml of fresh medium. Finally, the cell suspension was stored at -80°C in aliquots of 400µl until needed. For each experiment, an aliquot was thaw to avoid repeating freezing and thawing cycles.

### 1.2 Culture conditions and methods used to monitor growth

*M. hyopneumoniae* grows in suspension. This growth feature hampered the implementation of a high-throughput method to monitor growth in a 96-well plate format that was previously established for *M. pneumoniae* (EP application EP19382930.6). In particular, we found that culture conditions in this platform were not optimal for growing *M. hyopneumoniae*, even using shaking conditions. In addition, determination of cell biomass gain by protein quantification resulted in high background signals, especially from the 96-well plate format. For all these reasons, to estimate growth and aid the optimization process of a serum-free medium for *M. hyopneumoniae,* the following culture conditions and methods were set up. For each experiment, 3 culture replicates of 4ml each containing the tested medium were inoculated with frozen stocks (1:200 dilutions). For media comparison, modified Friss rich medium was used in each experiment as the reference medium (also tested by triplicate). Cultures were incubated at 37°C in orbital shaking (180rpm) in 15ml conical tubes and total DNA biomass was estimated after 96h of culture as follows. The 4ml bacterial culture was harvested by centrifugation (14100g, 10 min), and the pellet directly lysed with 300µl of Tissue and Cell Lysis solution plus Proteinase K from the MasterPure DNA purification Kit (Epicentre). DNA was extracted following the recommendations of the Kit manufacturer, and DNA measured using a fluorometric method (Qubit dsDNA HS assay Kit, Invitrogen).

To evaluate in more detail the performance of certain media formulations, growth curve analysis was performed as follows. Erlenmeyer flasks of 125ml containing 30 ml of medium were inoculated by triplicate with frozen stocks (1:200 dilutions) and incubated at 37°C in an orbital shaker set at 180rpm. Then, 1 ml of culture sample was collected at different time points (0h, 24h, 48h, 72, 96h, 168h), and cells harvested by centrifugation (14100g, 10 min) prior to DNA quantification following the methods described above.

### 1.3 Medium optimization workflow to develop a serum-free medium for M. hyopneumoniae.

A culture medium for mycoplasma likely requires a carbon source, amino acids, nucleosides, lipids, vitamins and co-factors, essential metals and minerals.. In this invention, we developed a culture medium free of animal serum, which was optimized for cultivation of *M. hyopneumoniae.* As a starting point to achieve this objective, we used a medium composition free of animal components, previously established for *M*. *pneumoniae* cultivation (EP application EP19382930.6). Primarily, this medium (named vB13, see Figure 1 for detailed composition) contains RNA, Yeastolate and the commercially available CMRL-1066 medium (Invitrogen), providing most of the amino acids, bases, vitamins and inorganic salts required for growth. As lipid sources, it contains phosphatidylcholine (PC), sphingomyelin (SPM), and cholesterol delivered by cyclodextrin. Of note, this medium supports growth of *M. pneumoniae,* allowing biomass yields close to 60-70% as compared to rich media.

As shown in Figure 2, the composition of the vB13 medium resulted sub-optimal for cultivation of *M. hyopneumoniae.* To optimize the composition of this medium for this specie, a workflow method to systematically test different media formulations was stablished (Fig. 3). First, the contribution of different concentrations of the original components in the performance of vB13 medium was tested. Then, it was tested the contribution of new components that could improve performance based on the review of the literature and the analysis of the metabolic map of *M. hyopneumoniae* (Kamminga et al., 2017). When an improvement was found, it was fixed, the influence of different concentrations of the other components was tested again on a systematic manner, as it was found that small variations could affect the optimal range of the other components. This methodology was repeated until it was not possible to find a significant improvement, resulting in a total of more than 170 experiments. For each media tested, growth was assessed by measuring DNA biomass at 96h of culture as detailed above (see method section entitled *"culture conditions and methods used to monitor growth"*)*.*

### 1.4 Major improvements found during medium optimization process.

Below, it is disclosed a summary of the major improvements found during the optimization process aimed to develop a serum-free culture medium for cultivation of *M. hyopneumoniae.* The composition details and performance of intermediate media versions is summarized in Figure 1.

### Buffering system

Although *M. hyopneumoniae* uses glucose as a carbon source and has the ability to ferment glucose, its metabolism produces less lactic acid compared to *M. pneumoniae,* resulting in a reduction of medium acidification. As a consequence, it was found that removal of HEPES from vB13 as a buffering system allowed us to better assess qualitative growth by monitoring color change without affecting growth rates.

### Carbon source

As previously described (Kamminga et al., 2017), it was found that pyruvate in addition to glucose improved growth of *M. hyopneumoniae.* Of note, 4 g/L instead of 2.2g/L of pyruvate was preferred in the present culture conditions (Fig. 4A). In addition, it was found that glycerol play a comparatively minor role in contrast to *M. pneumoniae.* Indeed, higher amounts of glycerol resulted in non-reproducible cultures.

### Amino acids and broth extracts

All the essential amino acids were provided by the commercially available CMRL-1066 medium (Invitrogen). Only glutamine was added separately as this amino acid is absent in CMRL. As in *M. pneumoniae,* increasing amino acids amounts did not improve growth, but the presence of peptones or similar hydrolyzed extracts were critical. In this regard, absence of TC Yeastolate (used here as it is not derived from animal materials) resulted in failure of growth (Fig. 4B). In comparison to vB13, it was found that a decrease of Yeastolate concentration from 10mg/L to 7.5 mg/L improved growth. Although other concentrations tested were tolerated, 7.5 mg/L seem to be optimal for growth in the context of the medium composition tested.

### Bases and nucleosides

Essential nucleosides were mostly provided by the CMRL-1066 medium and Yeastolate, although supplementation of extra nucleosides, especially thymidine improved slightly the robustness of the medium. Of note, *M. hyopneumoniae* lacks the thymidylate synthase in contrast to *M. pneumoniae.* The presence of RNA did not provide a better growth performance, and therefore it was removed from the original medium.

### Vitamins

Essential vitamins were mostly provided by the CMRL-1066 medium, except for thioctic acid which was supplemented separately. In *M. pneumoniae,* extra amounts of vitamins such as pyridoxamine, nicotinic acid, riboflavin and choline slightly improved the robustness of the medium. In contrast, the addition of these extra vitamins resulted in reduced yields in *M. hyopneumoniae* (Fig 4A).

### CMRL as a nutrient substitute

Commercially available CMRL-1066 medium was used to facilitate media preparation and substitute several components that should be otherwise supplemented separately, including for example amino acids, nucleosides, essential minerals, anti-oxidants, vitamins and other co-factors. The presence of CMRL in the medium is essential to support growth (Fig. 4B), and as in *M. pneumoniae,* it was found particularly important the adjustment of the amount of CMRL supplemented, being 0.5X the optimal concentration. Note that higher concentrations had inhibitory effects. Although the use of CMRL extremely simplify the preparation of the medium, it is also possible to add separately the components, since the formulation of CMRL is publically available.

### Lipids and lipid carriers

The delivery of lipids was critical for medium development, as variations in the lipid content resulted in the major improvements achieved. As in the case of *M. pneumoniae,* the cholesterol amount present in CMRL was not enough to support growth, demanding additional cholesterol supplementation with an optimal concentration close to 30µg/ml. In addition, cholesterol had to be delivered by a carrier protein such as BSA (Fig. 4A), as its replacement by cyclodextrin resulted in failure of growth. Similar results were obtained with PVA. The addition of liposomes or related lipid carriers may be capable to substitute BSA to obtain a full animal component-free medium. BSA concentrations around 0.5% appeared to provide optimal results under our culture conditions. It was also found that BSA complexed with a lipid mix containing palmitic acid, oleic acid and cholesterol resulted in decreased performance compared to BSA complexed with cholesterol alone (Fig. 4A).

Addition of phospholipids were also found fundamental to improve growth performance. In particular, phosphatidylcholine (PC) was absolutely required for growth (Fig. 7B), with concentrations ranging from 40 to 60 µg/ml being optimal. This was in contrast to *M. pneumoniae,* in which PC was an important growth factor, but not essential. Moreover, it was found that sphingomyelin (SPM) was less essential compared to PC. Although absence of SPM decreases growth significantly (Fig. 4B), higher amounts than 10 µg/ml were toxic in the context of the medium composition tested. Thus, decrease of SPM from 40 µg/ml to 10 µg/ml resulted in enhanced performance (Fig. 4A). Finally, it was found that addition of 10 to 20 µg/ml of L-α-Phosphatidyl-DL-glycerol and 1,2-Dipalmitoyl-sn-glycero-3-phosphocholine also improved growth (Fig. 7A).

### 1.5 Growth curve analyses and passaging experiments to test medium performance

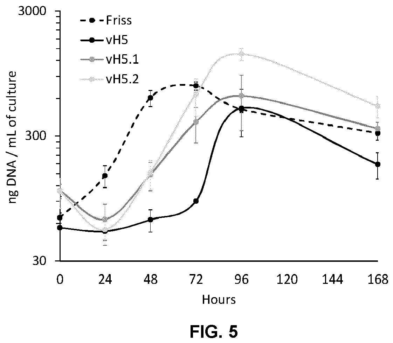

The optimization process resulted in media formulations reaching 60 to 80% of the DNA biomass obtained with rich medium (Figure 5). To better assess growth performance, growth curve analyses of the vH5 derivative mediums (see Figure 1 for detailed composition) was performed by measuring DNA biomass over time as described above (for details see method section entitled *"culture conditions and methods used to monitor growth"*)*.* Of note, cultures were scaled up to 30ml in contrast to the 4ml used in the optimization process. As shown in Figure 8, the enhanced performance of vH5.1 and especially vH5.2 versions compared to vH5 was reproduced. In particular, both vH5.1 and vH5.2 media formulations promoted growth with shorter lag phases, thus approximating to the yields obtained in rich medium after 72-96h of growth.

To test whether these media formulations could support growth after serial passages, passaging experiments were also performed as follows. Conical tubes of 15ml containing 4ml of medium were inoculated and incubated at 37°C in an orbital shaker set at 180rpm for 72h. For the next passage, 400µl of cell suspension was diluted in 4ml of fresh medium to start a new culture. This process could be repeated up to 2 passages. Based on these results, it was conclude that the media formulations of the vH5 derivative versions could be used for bacterial production upon the process of industrial scaling up.

## Claims

1. A serum-free culture medium suitable for the culture of *Mycoplasma hyopneumoniae* that comprises:
(i) a carbon source comprising at least a pyruvate salt,
(ii) an amino acid mixture,
(iii) a vitamin mixture,
(iv) lipids,
(v) inorganic salts,
(vi) a nucleoside mixture comprising thymidine,
(vii) a mixture of enzymatic cofactors,
(viii) a proteolytic digest of a microorganism or of a plant or animal tissue and
(ix) a vehicle to solubilize and deliver the lipids.

2. The medium of claim 1 wherein the carbon source comprises glucose, mannose, glycerol, acetate, glucuronate, , acetate, ascorbate and lactate or a combination thereof.

3. The medium according to claims 1 o 2 wherein the pyruvate is provided as the sodium salt and is found at a concentration of at least 2.2 g/L, preferably at least 4 g/L.

4. The medium of any preceding claim wherein the content in L-Glutamine is of least 2 mM.

5. The medium according to any preceding claim wherein the lipids are cholesterol, sphingomyelin, phosphatidylcholine, L-α-Phosphatidyl-DL-glycerol, 1,2-Dipalmitoyl-sn-glycero-3-phosphocholine or a combination thereof.

6. The medium according to claim 5 wherein the cholesterol is present at a concentration of about 30 µg/mL, wherein the sphingomyelin is present at a concentration of less than about 10 µg/mL, wherein the phosphatidylcholine is present at a concentration of ca. 40 µg/mL, preferably at ca. 60 µg/mL. wherein the L-α-Phosphatidyl-DL-glycerol is present at a concentration of between. 10 and 20 µg/mL and/or wherein the 1,2-Dipalmitoyl-sn-glycero-3-phosphocholine is present at a concentration of 10 to. 20 µg/mL.

7. The medium of any preceding claim wherein the proteolytic digest of a microorganism or of a plant or animal tissue is yeastolate.

8. The method of claim 7 wherein the yeastolate is present at a concentration of about 7,5 mg/L.

9. The medium according to any preceding claim wherein the vehicle to solubilize and deliver the lipids is selected from the group of a lipid-binding protein, PVA and a liposome.

10. The medium according to any preceding claim wherein the vehicle to solubilize and deliver the lipids is a lipid-binding protein, preferably delipidated BSA.

11. The medium according to claim 10 wherein the delipidated bovine serum albumin is complexed to cholesterol and not complexed with fatty acids.,

12. The medium according to any of claim 10 or 11 wherein the BSA is found at a concentration of 0,5% (w/w) or less, preferably at 0,3% (w/w) or less.

13. The medium according to any preceding aspect wherein the medium lacks a buffering system.

14. The medium according to any preceding claim wherein the components defined in the "component" column in the following table are present at concentrations indicated in the "Concentration" column.
| **Components** | **Concentration (mg/L)** |
|---|---|
| Glycine | 25.0 |
| Hydroxy L-proline | 5.0 |
| L-Alanine | 12,5 |
| L-Arginine hydrochloride | 35.0 |
| L-Aspartic acid | 15.0 |
| L-Cysteine | 99,94 |
| L-Cystine | 10.0 |
| L-Glutamic Acid | 37.5 |
| L-Histidine hydrochloride-H2O | 10.0 |
| L-Isoleucine | 10.0 |
| L-Leucine | 30.0 |
| L-Lysine hydrochloride | 35.0 |
| L-Methionine | 7.5 |
| L-Phenylalanine | 12.5 |
| L-Proline | 20.0 |
| L-Serine | 12.5 |
| L-Threonine | 15.0 |
| L-Tryptophan | 5.0 |
| L-Tyrosine | 20.0 |
| L-Valine | 12.5 |
| Ascorbic Acid | 25.0 |
| Biotin | 0.005 |
| Cholesterol | 0.1 |
| Choline chloride | 0.25 |
| D-Calcium pantothenate | 0.005 |
| Folic Acid | 0.005 |
| Niacinamide | 0.0125 |
| Nicotinic acid (Niacin) | 0.0125 |
| Para-Aminobenzoic Acid | 0.025 |
| Pyridoxal hydrochloride | 0.0125 |
| Pyridoxine hydrochloride | 0.0125 |
| Riboflavin | 0.005 |
| Thiamine hydrochloride | 0.005 |
| i-Inositol | 0.0025 |
| Calcium Chloride (CaCl2-2H2O) | 132.0 |
| Magnesium Sulfate (MgSO4-7H2O) | 100.0 |
| Potassium Chloride (KCl) | 200.0 |
| Sodium Bicarbonate (NaHCO3) | 1100.0 |
| Sodium Chloride (NaCl) | 3399.5 |
| Sodium Phosphate monobasic (NaH2PO4-2H2O) | 75.4 |
| 2'Deoxyadenosine | 5.0 |
| 2'Deoxycytidine | 5.0 |
| 2'Deoxyguanosine | 5.0 |
| 5-Methyl-deoxycytidine | 0.05 |
| Co-carboxylase | 0.05 |
| Coenzyme A | 1.25 |
| D-Glucose (Dextrose) | 500.0 |
| Diphosphopyridine nucleotide (NAD) | 3.5 |
| FAD (flavin adenine dinucleotide) | 0.05 |
| Glutathione (reduced) | 5.0 |
| Phenol Red | 10.0 |
| Sodium acetate-3H2O | 41.5 |
| Sodium glucuronate-H2O | 2.1 |
| Thymidine | 5.0 |
| Triphosphopyridine Nucleotide (NADP) | 0.5 |
| Tween 80® | 2.5 |
| Uridine 5'- triphosphate | 0.5 |

15. The medium according to claim 14 wherein the content of one or more components selected from the group consisting of Tween80, thiamin, niacinamide, nicotinic acid, folic acid, pathonthenic acid, ascorbic acid, riboflavin, spermidine, Para-Aminobenzoic Acid, Pyridoxal hydrochloride, Pyridoxine hydrochloride, Histidine, Methionine, Proline, Cysteine and Tryptophan is not higher than those provided in the Table in claim 36.

16. The medium according to any preceding claim wherein the medium is selected from the group consisting of the vH5, vH5.1 or vH5.2 as shown in Fig, 5.

17. A method for the culture of a Mycoplasma strain comprising placing an inoculum of said strain into the media according to any of claims 1 to 15 and maintaining the inoculated media under conditions adequate for the proliferation of the Mycoplasma strain.

18. A method for obtaining a biomass of a Mycoplasma strain which comprises placing an inoculum of said Mycoplasma strain into the media according to any of claims 1 to 15 and maintaining the inoculated media under conditions adequate for the proliferation of the Mycoplasma strain and the formation of the biomass.

19. The method according to any of claims 17 or 18 wherein the Mycoplasma is *Mycoplasma hyopenumoniae.*
